# NEW EUROPEAN PATENT SPECIFICATION

(11) **EP 1 189 881 B2**
(45) Date of publication and mention of the opposition decision: **23.01.2013**
(45) Mention of the grant of the patent: 29.09.2004
(21) Application number: 00930450.2
(22) Date of filing: 05.05.2000
(51) Int. Cl.: C07D 405/12, C07D 213/64, C07D 213/89, C07D 409/14

(54) **PROPANOIC ACID DERIVATIVES THAT INHIBIT THE BINDING OF INTEGRINS TO THEIR RECEPTORS**
PROPANSÄUREDERIVATE, DIE DIE BINDUNG VON INTEGRINEN AN IHRE REZEPTOREN BEHINDERN
DERIVES DE L'ACIDE PROPANOIQUE INHIBANT LA LIAISON DES INTEGRINES A LEURS RECEPTEURS

(30) Priority: 07.05.1999 US 132967 P; 10.12.1999 US 170441 P
(43) Date of publication of application: 27.03.2002
(73) Proprietor: ENCYSIVE PHARMACEUTICALS, INC., New York, NY 10017 (US)
(72) Inventor: BIEDIGER, Ronald, J., Houston, TX 77095 (US); HOLLAND, George, W., North Caldwell, NJ 07006 (US); KASSIR, Jamal, M., Houston, TX 77054 (US); LI, Wen, Houston, TX 77057 (US); MARKET, Robert, V., Pearland, TX 77581 (US); SCOTT, Ian, L., Delanson, NY 12053 (US); WU, Chengde, Pearland, TX 77584 (US)
(74) Representative: Weber, Martin
(86) International application number: PCT/US2000/012464
(87) International publication number: WO 2000/068188

(56) References cited:
- EP-A- 0 512 831
- EP-A- 0 761 680
- EP-A- 0 842 943
- EP-A- 0 903 353
- WO-A-00/21920
- WO-A-00/61631
- WO-A-98/04247
- WO-A-98/08840
- WO-A-98/16502
- WO-A-98/50420
- US-A- 5 847 135
- US-A- 5 874 424
- GOLEC J M C ET AL: "Structure-based design of non-peptidic pyridone aldehydes as inhibitors of interleukin-1beta converting enzyme" BIOORGANIC & MEDICINAL CHEMISTRY LETTERS, OXFORD, GB, vol. 7, no. 17, 9 September 1997 (1997-09-09), pages 2181-2186, XP004136410 ISSN: 0960-894X
- SEMPLE G ET AL: "Peptidomimetic aminomethylene ketone inhibitors of interleukin-1beta-converting enzyme (ICE)" BIOORGANIC & MEDICINAL CHEMISTRY LETTERS, OXFORD, GB, vol. 8, no. 8, 21 April 1998 (1998-04-21), pages 959-964, XP004136999 ISSN: 0960-894X
- SEMPLE G ET AL: "PYRIDONE-BASED PEPTIDOMIMETIC INHIBITORS OF INTERLEUKIN-1beta-CONVER TING ENZYME (ICE)" BIOORGANIC & MEDICINAL CHEMISTRY LETTERS, OXFORD, GB, vol. 7, no. 10, 20 May 1997 (1997-05-20), pages 1337-1342, XP004136329 ISSN: 0960-894X
- CHEMICAL ABSTRACTS, vol. 129, 28 May 1998, Columbus, Ohio, US; abstract no. 41414, AUDIA J. ET AL.: 'Preparation of N-(phenylacetyl)di and tripeptide derivatives for inhibiting amyloid peptide release' & WO 98 22494 A2 1998

## Description

This invention is directed generally to the inhibition of the binding of α₄β₁ integrin to its receptors, for example VCAM-1 (vascular cell adhesion molecule-1) and fibronectin. The invention also relates to compounds that inhibit this binding, to pharmaceutically active compositions comprising such compounds and to the use of such compounds either as above, or in formulations for the control or prevention of disease states in which α₄β₁ is involved.

### Background of the Invention

When a tissue has been invaded by a microorganism or has been damaged, white blood cells, also called leukocytes, play a major role in the inflammatory response. One of the most important aspects of the inflammatory response involves the cell adhesion event. Generally, white blood cells are found circulating through the bloodstream. However, when a tissue is infected or becomes damaged, the white blood cells recognize the invaded or damaged tissue, bind to the wall of the capillary and migrate through the capillary into the affected tissue. These events are mediated by a family of proteins called cell adhesion molecules.

There are three main types of white blood cells: granulocytes, monocytes and lymphocytes. The integrin α₄β₁ (also called VLA-4 for very late antigen-4) is a heterodimeric protein expressed on the surface of monocytes, lymphocytes and two subclasses of granulocytes: eosinophils and basophils. This protein plays a key role in cell adhesion through its ability to recognize and bind VCAM-1 and fibronectin, proteins associated with the endothelial cells that line the interior wall of capillaries.

Following infection or damage of tissue surrounding a capillary, endothelial cells express a series of adhesion molecules, including VCAM-1, that are critical for binding the white blood cells that are necessary for fighting infection. Prior to binding to VCAM-1 or fibronectin, the white blood cells initially bind to certain adhesion molecules to slow their flow and allow the cells to "roll" along the activated endothelium. Monocytes, lymphocytes, basophils and eosinophils are then able to firmly bind to VCAM-1 or fibronectin on the blood vessel wall via the α₄β₁ integrin. There is evidence that such interactions are also involved in transmigration of these white blood cells into the damaged tissue, as well as the initial rolling event itself.

Although white blood cell migration to the site of injury helps fight infection and destroy foreign material, in many instances this migration can become uncontrolled, with white blood cells flooding to the scene, causing widespread tissue damage. Compounds capable of blocking this process, therefore, may be beneficial as therapeutic agents. Thus, it would be useful to develop inhibitors that would prevent the binding of white blood cells to VCAM-1 and fibronectin.

Some of the diseases that might be treated by the inhibition of α₄β₁ binding include, but are not limited to, atherosclerosis, rheumatoid arthritis, asthma, allergy, multiple sclerosis, lupus, inflammatory bowel disease, graft rejection, contact hypersensitivity, and type I diabetes. In addition to being found on some white blood cells, α₄β₁ is also found on various cancer cells, including leukemia, melanoma, lymphoma and sarcoma cells. It has been suggested that cell adhesion involving α₄β₁ may be involved in the metastasis of certain cancers. Inhibitors of α₄β₁ binding may, therefore, also be useful in the treatment of some forms of cancer.

The isolation and purification of a peptide which inhibits the binding of α₄β₁ to a protein is disclosed in U.S. Patent No. 5,510,332. Peptides which inhibit binding are disclosed in WO 95/15973, EP 0 341 915, EP 0 422 938 A1, EP 0 842 943, U.S. Patent No. 5,192,746 and WO 96/06108. Novel compounds which are useful for inhibition and prevention of cell adhesion and cell adhesion-mediated pathologies are disclosed in WO 96/22966, WO 98/04247 and WO 98/04913.

It is therefore an object of the invention to provide novel compounds which are inhibitors of α₄β₁ binding, and pharmaceutical compositions including such novel compounds.

### Brief Summary of the Invention

The invention is directed to compounds of the following Formula : 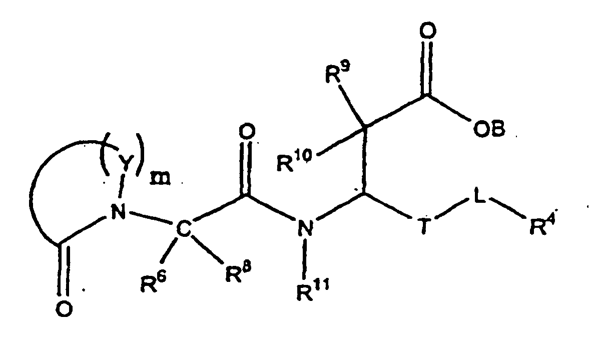 wherein the ring including Y is a mono-cyclic heterocycle consisting of an optionally substituted oxo-pyridinyl of the formula IV: 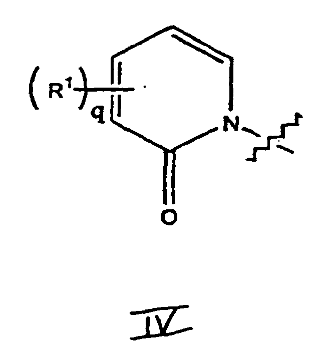
- q: is an integer of zero to four; and
- T: is selected from the group consisting of (CH₂)_{b} wherein b is an integer of 0 to 3;
- L: is selected from the group consisting of O, NR¹³, S, and (CH₂)ₙ wherein n is an integer of 0 or 1; and
- B, R¹, R⁴, R⁶, R⁹, R¹⁰, R¹¹ and R¹³: are independently selected from the group consisting of hydrogen, halogen, hydroxyl, alkyl alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), -NHC(O)NH(C₁-C₃ alkyl), -NHC(O)N(C₁-C₃ alkyl)C(O)NH(C₁-C₃ alkyl), -C₁-C₃ alkylamino, alkenylamino, alkynylamino, di(C₁-C₃ alkyl)amino, -C(O)O-(C₁-C₃ alkyl), -C(O)NH-(C₁-C₃ alkyl), -CH=NOH, -PO₃H₂, -OPO₃H₂, -C(O)N(C₁-C₃ alkyl)₂, haloalkyl, alkoxyalkoxy, carboxaldehyde, carboxamide, cycloalkyl cycloalkenyl, cycloalkynyl, cycloalkylalkyl, aryl, aroyl, aryloxy, arylamino, biaryl, thioaryl, diarylamino, heterocyclyl, alkylaryl aralkenyl, aralkyl, alkylheterocyclyl, heterocyclylalkyl, sulfonyl, -SO₂-(C₁-C₃ alkyl), -SO₃-(C₁-C₃ alkyl), sulfonamido, aryloxyalkyl, carboxyl, carbamate and -C(O)NH(benzyl);
- R⁸,: is independently selected from the group consisting of halogen, hydroxyl, alkyl, alkenyl alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), -NHC(O)NH(C₁-C₃ alkyl), -NHC(O)N(C₁-C₃ alkyl)C(O)NH(C₁-C₃ alkyl), -C₁-C₃ alkylamino, alkenylamino, alkynylamino, di(C₁-C₃ alkyl)amino, -C(O)O-(C₁-C₃ alkyl), -C(O)NH-(C₁-C₃ alkyl), -CH=NOH, -PO₃H₂, -OPO₃H₂, -C(O)N(C₁-C₃alkyl)₂, haloalkyl, alkoxyalkoxy, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, aryloxyalkyl, carboxyl, carbamate and -C(O)NH(benzyl);
wherein when L is -NR¹³-, R⁴ and R¹³ taken together may form a ring;
and wherein R⁶ and R⁸ taken together may form a ring;
and wherein R⁹ and R¹⁰ taken together may form a ring;
or a pharmaceutically acceptable salt thereof.

More specifically, the compounds of this invention may be described by Formula III below 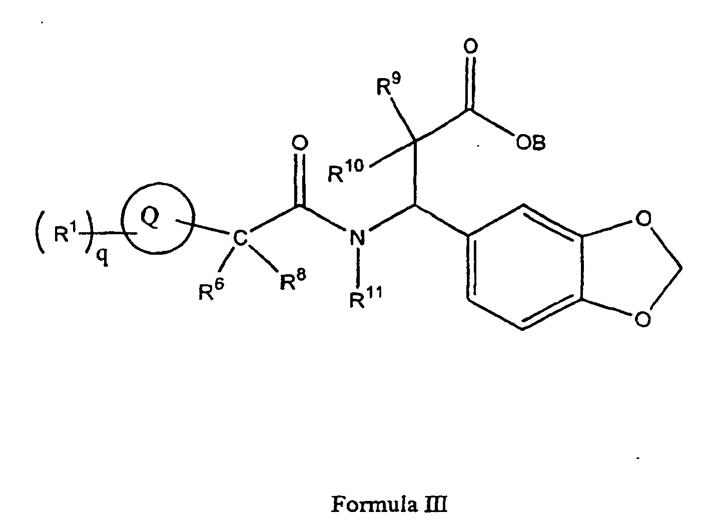 wherein circle Q is a ring consisting of 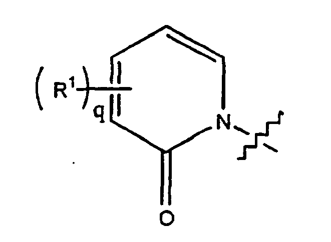
q is an integer of zero to four; and,
B, R¹, R⁶, R⁸, R⁹ , R¹⁰ and R¹¹ are each independently selected from the group consisting of hydrogen, halogen, hydroxyl, alkyl, alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), - NHC(O)NH(C₁-C₃ alkyl), -NHC(O)N(C₁-C₃ alkyl)C(O)NH(C₁-C₃ alkyl), -C₁-C₃ alkylamino, alkenylamino, alkynylamino, di(C₁-C₃ alkyl)amino, -C(O)O-(C₁-C₃ alkyl), -C(O)NH-(C₁-C₃ alkyl), - CH=NOH, -PO₃H₂, -OPO₃H₂, -C(O)N(C₁-C₃ alkyl)₂, haloalkyl, alkoxyalkoxy, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, aryloxyalkyl, carboxyl, carbamate and -C(O)NH(benzyl);
wherein R⁶ and R⁸ taken together may form a ring;
R⁹ and R¹⁰ taken together may form a ring;
or a pharmaceutically acceptable salt thereof.

Presently preferred compounds of Formula III may have R⁶, R⁸, R⁹ , R¹⁰ and R¹¹ each independently as hydrogen or alkyl, and R¹ at each occurrence, each independently as hydrogen, 2-thienylmethyl, benzyl or methyl. Presently preferred compounds include (3*S*)-3-(1,3-benzodioxol-5-yl)-3-((2*R*,*S*)-2-(3-benzyl-5-methyl-2-oxo-1(2*H*)-pyridinyl)hexanoylamino)propanoic acid, (3*S*)-3-(1,3-benzodioxol-5-yl)-3-((2*R*,*S*)-2-(3-(3-chlorobenzyl)-5-methyl-2-oxo-1(2*H*)-pyridinyl) hexanoylamino)propanoic acid, (3S)-3-(1,3-benzodioxol-5-yl)-3-(((2*S*)-2-(2-oxo-3-(phenylmethyl)-1(2H)-pyridinyl)hexanoyl)amino) propanoic acid, (3*S*)-3-(1,3-benzodioxol-5-yl)-3-((2--(3-cholorophenyl)methyl)-5-methyl-2-oxo-1(2H)-pyridinyl)hexanoyl)amino)propanoic acid and pharmaceutically acceptable salts thereof.

Derivatives of Formulae I, II and III which are esters, carbamates, and aminals are also contemplated.

The present invention also relates to pharmaceutical compositions comprising a physiologically acceptable diluent and at least one compound of the present invention.

The present invention further relates to a process of inhibiting the binding of α₄β₁ integrin to VCAM-1 comprising exposure of a cell expressing α₄β₁ integrin to a cell expressing VCAM-1 in the presence of an effective inhibiting amount of a compound of the present invention. The VCAM-1 may be on the surface of a vascular endothelial cell, an antigen presenting cell, or other cell type. The α₄β₁ may be on a white blood cell such as a monocyte, lymphocyte, granulocyte; a stem cell; or any other cell that naturally expresses α₄β₁.

The invention also provides a method for treating disease states mediated by α₄β₁ binding which comprises administration of an effective amount of a compound of the present invention, either alone or in formulation, to an afflicted patient.

### Detailed Description of the Invention

### Definition of Terms

The term "alkyl" as used herein, alone or in combination, refers to C₁-C₁₂ straight or branched, substituted or unsubstituted saturated chain radicals derived from saturated hydrocarbons by the removal of one hydrogen atom, unless the term alkyl is preceded by a Cₓ-C_{y} designation. Representative examples of alkyl groups include methyl, ethyl, n-propyl, iso-propyl, n-butyl, sec-butyl, iso-butyl, and tert-butyl among others.

The term "alkenyl" as used herein, alone or in combination, refers to a substituted or unsubstituted straight-chain or substituted or unsubstituted branched-chain alkenyl radical containing from 2 to 10 carbon atoms. Examples of such radicals include, but are not limited to, ethenyl, E- and Z-pentenyl, decenyl and the like.

The term "alkynyl" as used herein, alone or in combination, refers to a substituted or unsubstituted straight or substituted or unsubstituted branched chain alkynyl radical containing from 2 to 10 carbon atoms. Examples of such radicals include, but are not limited to ethynyl, propynyl, propargyl, butynyl, hexynyl, decynyl and the like.

The term "lower" modifying "alkyl", "alkenyl", "alkynyl" or "alkoxy" refers to a C₁-C₆ unit for a particular functionality. For example lower alkyl means C₁-C₆ alkyl.

The term "aliphatic acyl" as used herein, alone or in combination, refers to radicals of formula alkyl-C(O)-, alkenyl-C(O)- and alkynyl-C(O)- derived from an alkane-, alkene- or alkyncarboxylic acid, wherein the terms "alkyl", "alkenyl" and "alkynyl" are as defined above. Examples of such aliphatic acyl radicals include, but are not limited to, acetyl, propionyl, butyryl, valeryl, 4-methylvaleryl, acryloyl, crotyl, propiolyl and methylpropiolyl, among others.

The term "cycloalkyl" as used herein refers to an aliphatic ring system having 3 to 10 carbon atoms and 1 to 3 rings, including, but not limited to cyclopropyl, cyclopentyl, cyclohexyl, norbornyl, and adamantyl among others. Cycloalkyl groups can be unsubstituted or substituted with one, two or three substituents independently selected from lower alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide.

"Cycloalkyl" includes cis or trans forms. Furthermore, the substituents may either be in endo or exo positions in the bridged bicyclic systems.

The term "cycloalkenyl" as used herein alone or in combination refers to a cyclic carbocycle containing from 4 to 8 carbon atoms and one or more double bonds. Examples of such cycloalkenyl radicals include, but are not limited to, cyclopentenyl, cyclohexenyl, cyclopentadienyl and the like.

The term "cycloalkylalkyl" as used herein refers to a cycloalkyl group appended to a lower alhyl radical, including, but not limited to cyclohexylmethyl.

The term "halo" or "halogen" as used herein refers to I, Br, Cl or F.

The term "haloalkyl" as used herein refers to a lower alkyl radical, to which is appended at least one halogen substituent, for example chloromethyl, fluoroethyl, trifluoromethyl and pentafluoroethyl among others.

The term "alkoxy" as used herein, alone or in combination, refers to an alkyl ether radical, wherein the term "alkyl" is as defined above. Examples of suitable alkyl ether radicals include, but are not limited to, methoxy, ethoxy, n-propoxy, iso-propoxy, n-butoxy, iso-butoxy, sec-butoxy, tert-butoxy and the like.

The term "alkenoxy" as used herein, alone or in combination, refers to a radical of formula alkenyl-O-, provided that the radical is not an enol ether, wherein the term "alkenyl" is as defined above. Examples of suitable alkenoxy radicals include, but are not limited to, allyloxy, E- and Z- 3-methyl-2-propenoxy and the like.

The term "alkynoxy" as used herein, alone or in combination, refers to a radical of formula alkynyl-O-, provided that the radical is not an -ynol ether. Examples of suitable alkynoxy radicals include, but are not limited to, propargyloxy, 2-butynyloxy and the like.

The term "carboxyl" as used herein refers to a carboxylic acid radical, - C(O)OH.

The term "thioalkoxy" refers to a thioether radical of formula alkyl-S-, wherein "alkyl" is as defined above.

The term "carboxaldehyde" as used herein refers to -C(O)R wherein R is hydrogen.

The terms "carboxamide" or "amide" as used herein refer to -C(O)NRₐR_{b} wherein Rₐ and R_{b} are each independently hydrogen, alkyl or any other suitable substituent.

The term "carboxy" as used herein refers to -C(O)O-.

The term "alkoxyalkoxy" as used herein refers to R_{c}O-R_{d}O- wherein R_{c} is lower alkyl as defined above and R_{d} is alkylene wherein alkylene is -(CH₂)ₙ,- wherein n' is an integer from 1 to 6. Representative examples of alkoxyalkoxy groups include methoxymethoxy, ethoxymethoxy, t-butoxymethoxy among others.

The term "alkylamino" as used herein refers to RₑNH- wherein Rₑ is a lower alkyl group, for example, ethylamino, butylamino, among others.

The term "alkenylamino" as used herein, alone or in combination, refers to a radical of formula alkenyl-NH-or (alkenyl)₂N-, wherein the term "alkenyl" is as defined above, provided that the radical is not an enamine. An example of such alkenylamino radical is the allylamino radical.

The term "alkynylamino" as used herein, alone or in combination, refers to a radical of formula alkynyl-NH- or (alkynyl)₂N- wherein the term "alkynyl" is as defined above, provided that the radical is not an amine. An example of such alkynylamino radicals is the propargyl amino radical.

The term "dialkylamino" as used herein refers to R_{f}R_{g}N- wherein R_{f} and R_{g} are independently selected from lower alkyl, for example diethylamino, and methyl propylamino, among others.

The term "amino" as used herein refers to H₂N- .

The term "alkoxycarbonyl" as used herein refers to an alkoxyl group as previously defined appended to the parent molecular moiety through a carbonyl group. Examples of alkoxycarbonyl include methoxycarbonyl, ethoxycarbonyl, and isopropoxycarbonyl among others.

The term "aryl" or "aromatic" as used herein alone or in combination, refers to a substituted or unsubstituted carbocyclic aromatic group having about 6 to 12 carbon atoms such as phenyl, naphthyl, indenyl, indanyl, azulenyl, fluorenyl and anthracenyl; or a heterocyclic aromatic group which is an aromatic ring containing at least one endocyclic N, O or S atom such as furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl and the like. "Aralkyl" and "alkylaryl" employ the term "alkyl" as defined above. Rings may be multiply substituted.

The term "aralkyl" as used herein, alone or in combination, refers to an aryl substituted alkyl radical, wherein the terms "alkyl" and "aryl" are as defined above. Examples of suitable aralkyl radicals include, but are not limited to, phenylmethyl, phenethyl, phenylhexyl, diphenylmethyl, pyridylmethyl, tetrazolyl methyl, furylmethyl, imidazolyl methyl, indolylmethyl, thienylpropyl and the like.

The term "aralkenyl" as used herein, alone or in combination, refers to an aryl substituted alkenyl radical, wherein the terms "aryl" and "alkenyl" are as defined above.

The term "arylamino" as used herein, alone or in combination, refers to a radical of formula aryl-NH-, wherein "aryl" is as defined above. Examples of arylamino radicals include, but are not limited to, phenylamino(anilido), naphthlamino, 2-, 3-, and 4- pyridylamino and the like.

The term "biaryl" as used herein, alone or in combination, refers to a radical of formula aryl-aryl, wherein the term "aryl" is as defined above.

The term "thioaryl" as used herein, alone or in combination, refers to a radical of formula aryl-S-, wherein the term "aryl" is as defined above. An example of a thioaryl radical is the thiophenyl radical.

The term "aroyl" as used herein, alone or in combination, refers to a radical of formula aryl-CO-, wherein the term "aryl" is as defined above. Examples of suitable aromatic acyl radicals include, but are not limited to, benzoyl, 4-halobenzoyl, 4-carboxybenzoyl, naphthoyl, pyridylcarbonyl and the like.

The term "heterocyclyl" as used herein, alone or in combination, refers to a non-aromatic 3- to 10- membered ring containing at least one endocyclic N, O, or S atom. The heterocycle may be optionally aryl-fused. The heterocycle may also optionally be substituted with at least one substituent which is independently selected from the group consisting of hydrogen, halogen, hydroxyl, amino, nitro, trifluoromethyl, trifluoromethoxy, alkyl, aralkyl, alkenyl, alkynyl, aryl, cyano, carboxy, carboalkoxy, carboxyalkyl, oxo, arylsulfonyl and aralkylaminocarbonyl among others.

The term "alkylheterocyclyl" as used herein refers to an alkyl group as previously defined appended to the parent molecular moiety through a heterocyclyl group, including but not limited to 2-methyl-5-thiazolyl, 2-methyl-1-pyrrolyl and 5-ethyl-2-thiophenyl.

The term "heterocyclylalkyl" as used herein refers to a heterocyclyl group as previously defined appended to the parent molecular moiety through an alkyl group, including but not limited to 2-thienylmethyl, 2-pyridinylmethyl and 2-( 1-piperidinyl) ethyl.

The term "aminal" as used herein refers to a hemi-acetal of the structure RₕC(NRᵢRⱼ)(NRₖRₗ)- wherein Rₕ, Rᵢ, Rⱼ, Rₖ and Rₗ are each independently hydrogen, alkyl or any other suitable substituent.

The term "ester" as used herein refers to -C(O)Rₘ, wherein Rₘ is hydrogen, alkyl or any other suitable substituent.

The term "carbamate" as used herein refers to compounds based on carbamic acid NH₂C(O)OH.

Substitution may be by one or more groups such as alcohols, ethers, esters, amides, sulfones, sulfides, hydroxyl, nitro, cyano, carboxy, amines, heteroatoms, lower alkyl, lower alkoxy, lower alkoxycarbonyl, alkoxyalkoxy, acyloxy, halogens, trifluoromethoxy, trifluoromethyl, alkyl, aralkyl, alkenyl, alkynyl, aryl, cyano, carboxy, carboalkoxy, carboxyalkyl, cycloalkyl, cycloalkylalkyl, heterocyclyl, alkylheterocyclyl, heterocyclylalkyl, oxo, arylsulfonyl and aralkylaminocarbonyl or any of the substituents of the preceding paragraphs or any of those substituents either attached directly or by suitable linkers. The linkers are typically short chains of 1-3 atoms containing any combination of-C-, -C(O)-, -NH-, -S-, -S(O)-, -O-, -C(O)O- or -S(O)O-. Rings may be substituted multiple times.

The terms "electron-withdrawing" or "electron-donating" refer to the ability of a substituent to withdraw or donate electrons relative to that of hydrogen if hydrogen occupied the same position in the molecule. These terms are well-understood by one skilled in the art and are discussed in Advanced Organic Chemistry by J. March, 1985, pp. 16-18, incorporated herein by reference. Electron withdrawing groups include halo, nitro, carboxyl, lower alkenyl, lower alkynyl, carboxaldehyde, carboxyamido, aryl, quaternary ammonium, trifluoromethyl, and aryl lower alkanoyl among others. Electron donating groups include such groups as hydroxy, lower alkyl, amino, lower alkylamino, di(lower alkyl)amino, aryloxy, mercapto, lower alkylthio, lower alkylmercapto, and disulfide among others. One skilled in the art will appreciate that the aforesaid substituents may have electron donating or electron withdrawing properties under different chemical conditions. Moreover, the present invention contemplates any combination of substituents selected from the above-identified groups.

The most preferred electron donating or electron withdrawing substituents are halo, nitro, alkanoyl, carboxaldehyde, arylalkanoyl, aryloxy, carboxyl, carboxamide, cyano, sulfonyl, sulfoxide, heterocyclyl, guanidine, quaternary ammonium, lower alkenyl, lower alkynyl, sulfonium salts, hydroxy, lower alkoxy, lower alkyl, amino, lower alkylamino, di(lower alkyl)amino, amine lower alkyl mercapto, mercaptoalkyl, alkylthio and alkyldithio.

As used herein, the term "composition" is intended to encompass a product comprising the specified ingredients in the specified amounts, as well as any product which results, directly or indirectly, from a combination of the specified ingredients in the specified amounts.

The ring including Y in Formula II or ring Q in Formulae I and III can be a mono-cyclic heterocycle or aromatic ring, or can be a bicyclic ring. When more than one Y is C(R²)(R³), the C substituents from each Y may be joined to form a ring.

Suitable substituents for the aryl, alkyl, cycloalkyl, heterocyclyl groups or ring including Y defined above, when present, include alcohols, amines, heteroatoms, or any combination of aryl, alkyl, cycloalkyl or heterocyclyl groups either attached directly, or *via* suitable linkers. The linkers are typically short chains of 1-3 atoms containing any combination of C, C=O, CO₂, O, N, S, S=O, SO₂, as for example ethers, amides, amines, ureas, sulfamides, sulfonamides, and the like.

For example, R¹, R², R³, R⁵, R⁷, R¹¹ and R¹³ in Formulas I, II and III above may independently be, but are not limited to, phenyl, thienylmethyl, isobutyl, n-butyl, 2-thienylmethyl, 1,3-thiazol-2-yl-methyl, benzyl, thienyl, 3-pyridinylmethyl, 3-methyl-1-benzothiophen-2-yl, allyl, 3-methoxybenzyl, propyl, 2-ethoxyethyl, cyclopropylmethyl, benzylsulfanylmethyl, benzylsulfonylmethyl, phenylsulfanylmethyl, phenethylsulfanylmethyl, 3-phenylpropylsulfanylmethyl, 4-((2-toluidinocarbonyl)amino)benzyl, 2-pyridinylethyl, 2-(1H-indol-3-yl)ethyl, 1H-benzimidazol-2-yl, 4-piperidinylmethyl, 3-hydroxy-4-methoxybenzyl, 4-hydroxyphenethyl, 4-aminobenzyl, phenylsulfonylmethyl, 4-(acetylamino)phenyl, 4-methoxyphenyl, 4-aminophenyl, 4-chlorophenyl, (4-(benzylsulfonyl)amino)phenyl, (4-(methylsulfonyl)amino)phenyl, 2-aminophenyl, 2-methylphenyl, isopropyl, 2-oxo-1-pyrrolidinyl, 3-(methylsulfanyl)propyl, (propylsulfanyl)methyl, octylsulfanylmethyl, 3-aminophenyl, 4-((2-toluidinocarbonyl)amino)phenyl, 2-((methylbenzyl)amino)benzyl, methylsulfanylethyl, or ethylsulfanylmethyl. R⁶ and R⁸ may be linked to form a ring such as cyclopropyl, cyclobutyl, cyclopentyl, cyclohexyl, 4-piperidinyl, and 4-tetrahydropyranyl among others. R⁴ and R¹³ may be linked to form a ring such as pyrrolidino, 1-piperidino, 4-methyl-1-piperazino, 4-aceto-1-piperazino, and 4-morpholino among others. R⁹ and R¹⁰ may be linked to form a ring such as cyclopropyl, cyclobutyl, cyclopentyl, and cyclohexyl among others.

The R⁴ substituent for Formulae I and II above may be, but is not limited to, 1,3-benzodioxol-5-yl, 1-naphthyl, thienyl, 4-isobutoxyphenyl, 2,6-dimethylphenyl, allyloxyphenyl, 3-bromo-4-methoxyphenyl, 4-butoxyphenyl, 1-benzofuran-2-yl, 2-thienylmethyl, phenyl, methysulfanyl, phenylsulfanyl, phenethylsulfanyl, 4-bromo-2-thienyl, 3-methyl-2-thienyl, or 4,5-dihydro-1,3-oxazol-2-yl.

The R⁶ and R⁸ substituents for Formulas I, II and III above may be, but are not limited to hydrogen, butyl, benzyl, benzyloxymethyl, ethyl, propyl, phenylsulfanylmethyl, benzylsulfanylmethyl, methylsulfanylethyl, ethylsulfanylmethyl, methyl, or carboxyethyl.

### Abbreviations

Abbreviations which have been used in the schemes and the examples which follow are: BOC for t-butyloxycarbonyl; EtOAc for ethyl acetate; DMF for dimethylformamide; THF for tetrahydrofuran; Tos for p-toluenesulfonyl; DCC for dicyclohexylcarbodiimide; HOBT for 1-hydroxybenzotriazole; TFAA for trifluoroacetic anhydride; NMM for N-methyl morpholine; DIPEA for diisopropylethylamine; DCM for methylene dichloride; LHMDS for lithium hexamethyl disilazide; NaHMDS for sodium hexamethyl disilazide; CDI for 1,1'-carbonyldiimidazole HBTU for O-benzotriazol-1-yl-N,N,N',N'-tetramethyluronium hexafluorophosphate, EDCI for 1-[3-(dimethylamino) propyl]-3-ethylcarbodiimide hydrochloride and TBS for TRIS-buffered saline. Amino acids are abbreviated as follows: C for L-cysteine; D for L-aspartic acid; E for L-glutamic acid; G for glycine; H for L-histidine; I for L-isoleucine; L for L-leucine; N for L-asparagine; P for L-proline; Q for L-glutamine; S for L-serine; T for L-threonine; V for L-valine, and W for L-tryptophan.

Examples of the procedures utilized to synthesize the compounds are illustrated by the following schemes.

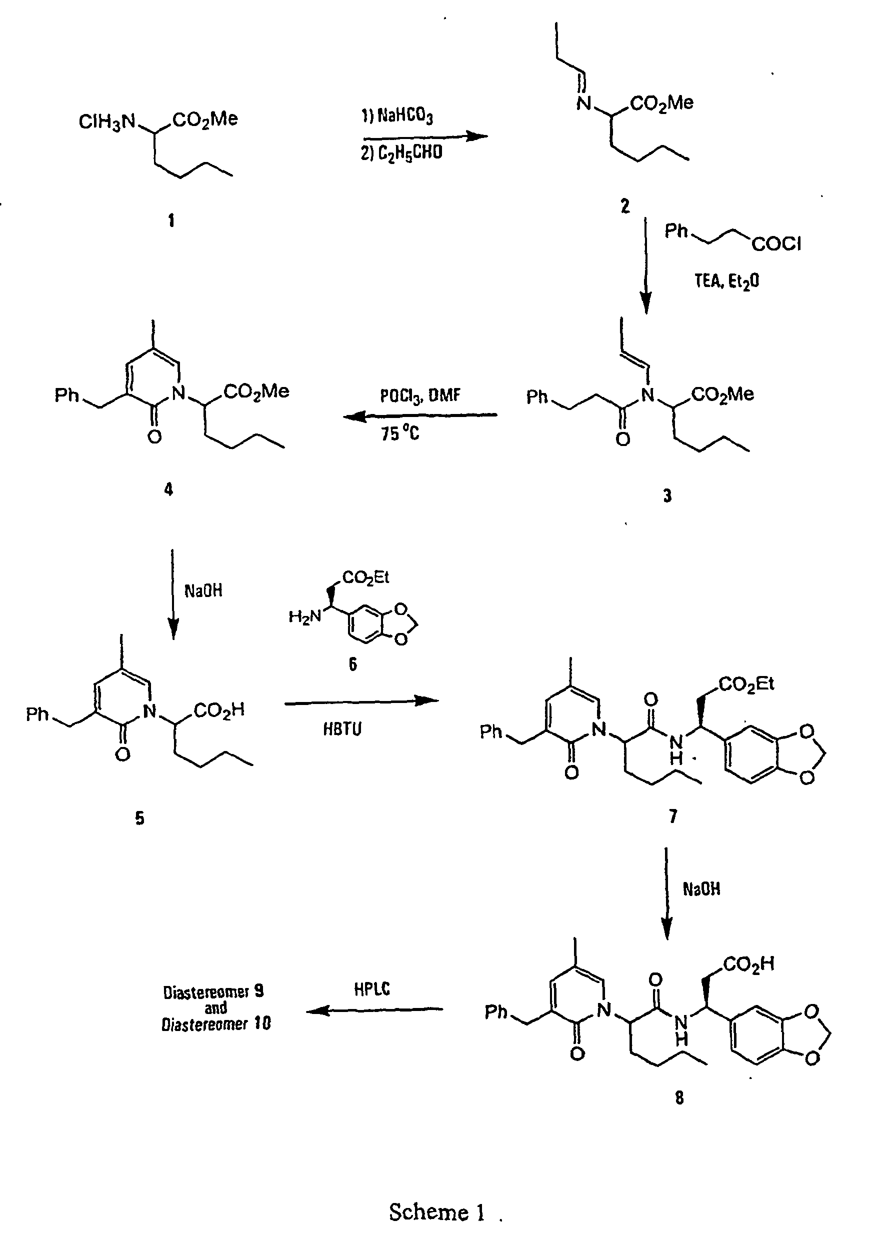

Scheme 3, shown below, illustrates the procedure described in Example 11.

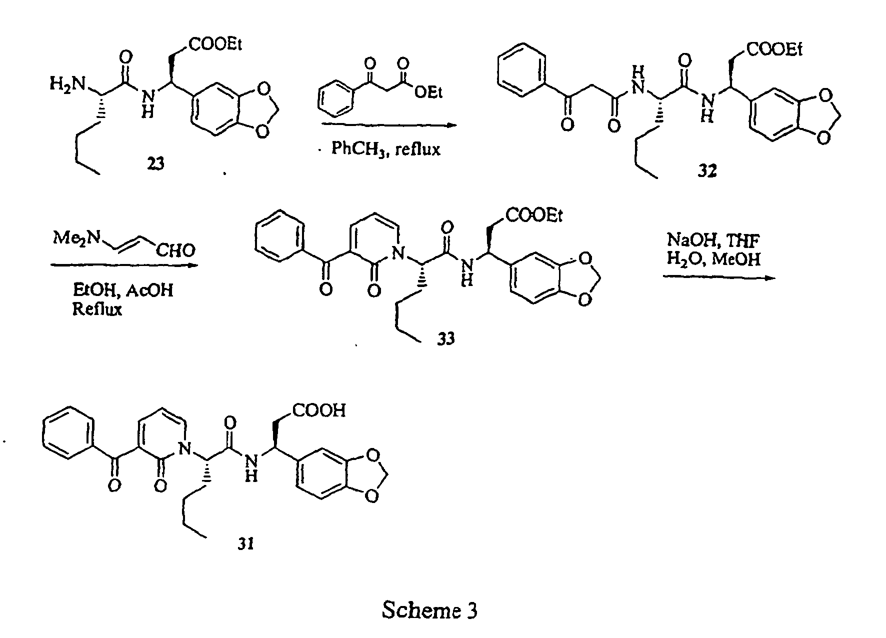

Scheme 4, shown below, illustrates Example 12.

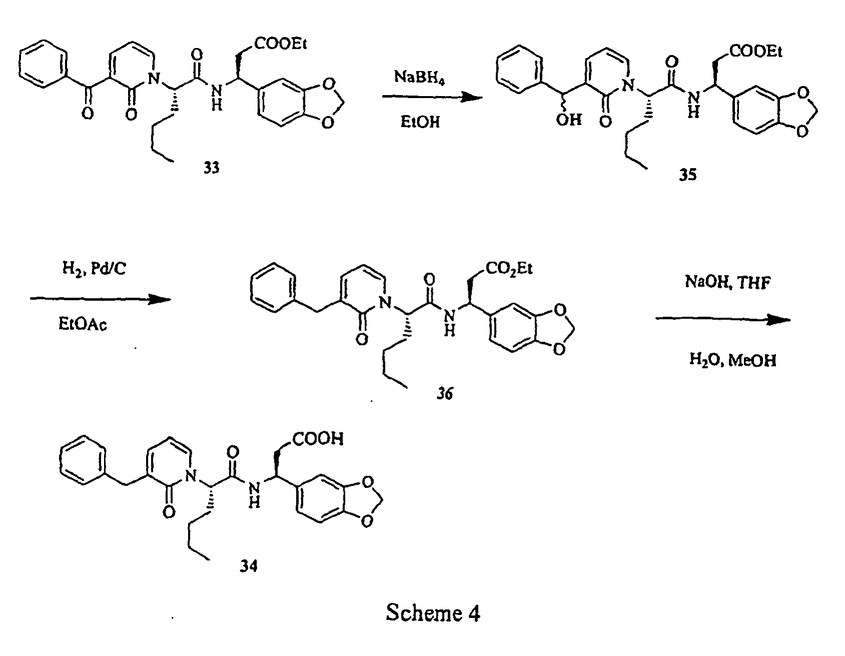

Scheme 5, shown below, illustrates the procedure of Example 13.

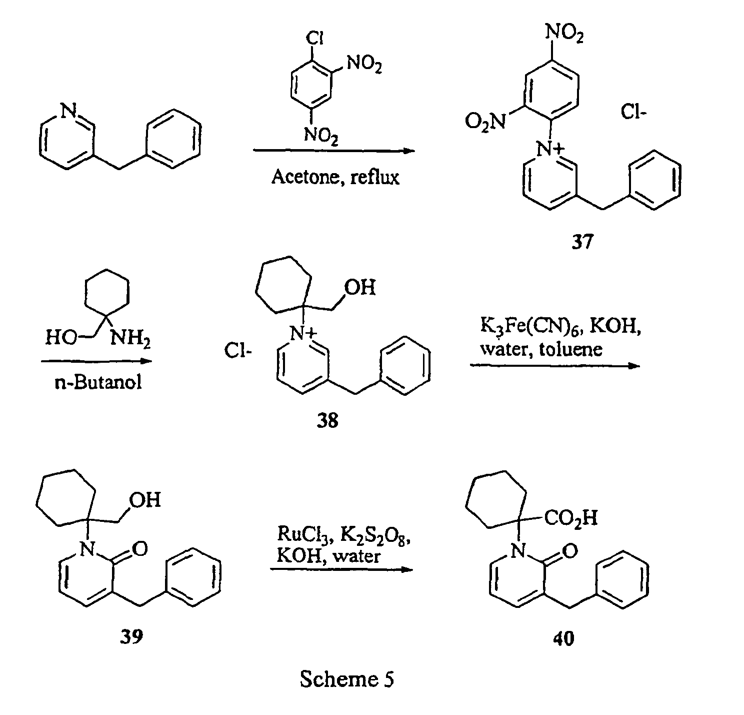

Scheme 6, shown below, illustrates the procedure described in Example 14.

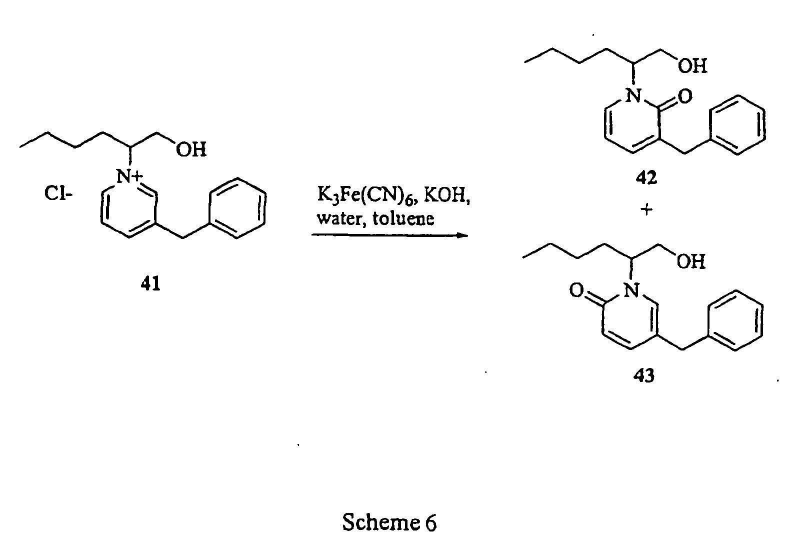

A detailed description of the preparation of representative compounds of the present invention is set forth in the Examples.

The compounds of the present invention can be used in the form of pharmaceutically acceptable salts derived from inorganic or organic acids. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge et al. describe pharmaceutically acceptable salts in detail in J. Pharmaceutical Sciences, 1977, 66: 1 et seq. The salts can be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quatemized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

Dosage forms for topical administration of a compound of this invention include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also contemplated as being within the scope of this invention.

Actual dosage levels of active ingredients in the pharmaceutical compositions of this invention can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

When used in the above or other treatments, a therapeutically effective amount of one of the compounds of the present invention can be employed in pure form or, where such forms exist, in pharmaceutically acceptable salt, ester or prodrug form. Alternatively, the compound can be administered as a pharmaceutical composition containing the compound of interest in combination with one or more pharmaceutically acceptable excipients. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or lower animal may range from about 0.0001 to about 1000 mg/kg/day. For purposes of oral administration, more preferable doses can be in the range of from about 0.001 to about 5 mg/kg/day. If desired, the effective daily dose can be divided into multiple doses for purposes of administration; consequently, single dose compositions may contain such amounts or submultiples thereof to make up the daily dose.

The present invention also provides pharmaceutical compositions that comprise compounds of the present invention formulated together with one or more non-toxic pharmaceutically acceptable carriers. The pharmaceutical compositions can be specially formulated for oral administration in solid or liquid form, for parenteral injection or for rectal administration.

The pharmaceutical compositions of this invention can be administered to humans and other mammals orally, rectally, parenterally, intracisternally, intravaginally, intraperitoneally, topically (as by powders, ointments or drops), bucally or as an oral or nasal spray. The term "parenterally," as used herein, refers to modes of administration which include intravenous, intramuscular, intraperitoneal, intrastemal, subcutaneous and intraarticular injection and infusion.

In another aspect, the present invention provides a pharmaceutical composition comprising a component of the present invention and a physiologically tolerable diluent. The present invention includes one or more compounds as described above formulated into compositions together with one or more non-toxic physiologically tolerable or acceptable diluents, carriers, adjuvants or vehicles that are collectively referred to herein as diluents, for parenteral injection, for intranasal delivery, for oral administration in solid or liquid form, for rectal or topical administration, or the like.

The compositions can also be delivered through a catheter for local delivery at a target site, via an intracoronary stent (a tubular device composed of a fine wire mesh), or via a biodegradable polymer. The compounds may also be complexed to ligands, such as antibodies, for targeted delivery.

Compositions suitable for parenteral injection may comprise physiologically acceptable, sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), vegetable oils (such as olive oil), injectable organic esters such as ethyl oleate, and suitable mixtures thereof.

These compositions can also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Proper fluidity can be maintained, for example, by the use of coating materials such as lecithin, by the maintenance of the required particle size in the case of dispersions and by the use of surfactants.
In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Compounds of the present invention may exist as stereoisomers wherein asymmetric or chiral centers are present. These stereoisomers are "R" or "S" depending on the configuration of substituents around the chiral carbon atom. The present invention contemplates various stereoisomers and mixtures thereof. Stereoisomers include enantiomers and diastereomers, and mixtures of enantiomers or diastereomers. Individual stereoisomers of compounds of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

The compounds of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention. In another aspect, the present invention contemplates a process of inhibiting the binding of α₄β₁ integrin to VCAM-1. A process of the present invention can be used either *in vitro* or *in vivo.* In accordance with a process of the present invention, a cell expressing α₄β₁ integrin is exposed to a cell expressing VCAM-1 in the presence of an effective inhibiting amount of a compound of the present invention.

A cell expressing α₄β₁ integrin can be a naturally occurring white blood cell, mast cell or other cell type that naturally expresses α₄β₁ on the cell surface, or a cell transfected with an expression vector that contains a poly-nucleotide (e.g., genomic DNA or cDNA) that encodes α₄β₁ integrin. In an especially preferred embodiment, α₄β₁ integrin is present on the surface of a white blood cell such as a monocyte, a lymphocyte or a granulocyte (e.g., an eosinophil or a basophil).

A cell that expresses VCAM-1 can be a naturally occurring cell (e.g. an endothelial cell) or a cell transfected with an expression vector containing a polynucleotide that encodes VCAM-1. Methods for producing transfected cells that express VCAM-1 are well known in the art.

Where VCAM-1 exists on the surface of cell, the expression of that VCAM-1 is preferably induced by inflammatory cytokines such as tumor necrosis factor-α, interleukin-4 and interleukin-1β.

Where the cells expressing α₄β₁ integrin and VCAM-1 are in a living organism, a compound of the present invention is administered in an effective amount to the living organism. Preferably, the compound is in a pharmaceutical composition of this invention. A process of the present invention is especially useful in treating diseases associated with uncontrolled migration of white blood cells to damaged tissue. Such diseases include, but are not limited to, asthma, atherosclerosis, rheumatoid arthritis, allergy, multiple sclerosis, lupus, inflammatory bowel disease, graft rejection, contact hypersensitivity, type I diabetes, leukemia, and brain cancer. Administration is preferably accomplished via intravascular, subcutaneous, intranasal, transdermal or oral delivery.

The present invention also provides a process of selectively inhibiting the binding of α₄β₁ integrin to a protein comprising exposing the integrin to the protein in the presence of an effective inhibiting amount of a compound of the present invention. In a preferred embodiment, the α₄β₁ integrin is expressed on the surface of a cell, either naturally occurring or a cell transformed to express α₄β₁ integrin.

The protein to which the α₄β₁ integrin binds can be expressed either on a cell surface or be part of the extracellular matrix. Especially preferred proteins are fibronectin or invasin.

The ability of compounds of the present invention to inhibit binding is described in detail hereinafter in the Examples. These Examples are presented to describe preferred embodiments and utilities of the invention and are not meant to limit the invention unless otherwise stated in the claims appended hereto.

### Example 1

Compound 8, (3*S*)-3-(1,3-benzodioxol-5-yl)-3-((2*R*,*S*)-2-(3-benzyl-5-methyl-2-oxo-1(2*H*)-pyridinyl)hexanoylamino)propanoic acid, of the structure shown below, was synthesized as follows. 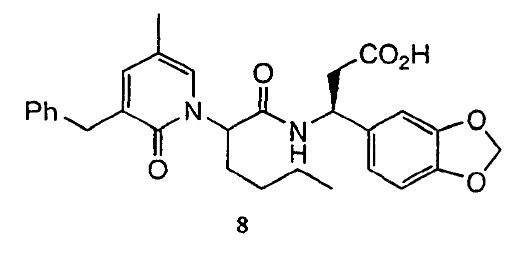 The structures of the compounds identified by number in this Example are found in Scheme 1 above.

Step 1: A solution of 540 mg of 2-aminohexanoic acid methyl ester hydrochloride salt **1** in 20 ml of methylene chloride was washed with excess saturated sodium bicarbonate. The organic layer was separated, dried over magnesium sulfate, and concentrated *in vacuo* to give 365 mg of 2-aminohexanoic acid methyl ester as a colorless oil. This material was combined with 5 ml of benzene, 0.28 ml of propionaldehyde, and excess magnesium sulfate. After stirring for 15 minutes, the reaction mixture was filtered and concentrated *in vacuo* to yield 420 mg of compound **2** as a colorless oil. Compound **2** was used directly without further purification.

Step 2: To an ice-bath cooled solution of 1050 mg of compound **2** in 10 ml of diethyl ether, under a positive nitrogen atmosphere, was added 0.80 ml of triethylamine, and a solution of 964 mg of 3-phenylpropanoyl chloride in 2 ml of diethyl ether. The ice bath was removed and the reaction mixture stirred for 30 minutes. The reaction mixture was then concentrated *in vacuo* and the residual materials further separated by silica gel chromatography using 15% ethyl acetate / hexane as the eluant to yield 468 mg of compound **3** as a colorless oil. Compound 3: ¹H NMR (CDCl₃): δ 0.87 (t, J = 7.0 Hz, 3H), 1.26 (m, 4H), 1.68 (dd, J = 7.0, 1.1 Hz, 3H), 1.74 (m, 1H), 1.97 (m, 1H), 2.70 (t, J = 7.9 Hz, 2H), 2.96 (t, J = 7.9 Hz, 2H), 3.68 (s, 3H), 4.96 (dd, J. = 10.1, 5.3 Hz, 1H), 5.32 (dq, J = 13.9, 7.0 Hz, 1H), 6.13 (dd, J = 13.9, 1.1 Hz, 1H), 7.20 (m, 2H), 7.25 (m, 3H).

Step 3: N,N-Dimethylformamide (1.63 ml) was added dropwise to an ice-cooled flask containing 4.57 ml of phosphorus oxychloride sealed under a positive nitrogen atmosphere. After 5 minutes, the reaction solution was cannulated into a flask containing 2.22 gm of compound **3**. This mixture was stirred at room temperature under a positive nitrogen atmosphere for 2 hours and then heated at 75 °C for 46 hours. The dark-colored reaction mixture was poured over ice and mixed with an excess of sodium bicarbonate and ethyl acetate. The mixture was saturated with sodium chloride and the organic layer separated. The aqueous layer was extracted (3 X 100 ml) with ethyl acetate. The combined organic materials were dried over magnesium sulfate and concentrated *in vacuo* to yield 1.70 gm of a dark-colored oil. Methylene chloride extraction (3 X ) of the aqueous layer yielded an additional 200 mg of material after drying (MgSO₄) and condensation *in vacuo*. The combined residual oils were further purified by silica gel chromatography using 20% -25% ethyl acetate / hexane as the eluant to yield 815 mg of compound **4** as a yellow oil. Compound 4: ¹H NMR (CDCl₃): δ 0.87 (t, J = 7.2 Hz, 3H), 1.18 (m, 1H), 1.31 (m, 3H), 1.87 (m, 1H), 2.00 (d, J = 0.7 Hz, 3H), 2.16 (m, 1H), 3.72 (s, 3H), 3.85 (br. s, 2H), 5.57 (dd, J = 10.1, 5.7 Hz, 1H), 6.82 (br. s, 1H), 6.94 (br, s, 1H), 7.23 (m, 3H), 7.30 (m, 2H).

Step 4: To a solution of 86 mg of compound **4** in 3 ml of tetrahydrofuran was added 1 ml of 2N sodium hydroxide and 2 ml of methanol. After complete hydrolysis, the reaction mixture was acidified with 2N hydrochloric acid and saturated with sodium chloride. The mixture was extracted (3X) with ethyl acetate and the combined extracts were dried with magnesium sulfate and concentrated *in vacuo* to yield 80 mg of compound 5 as a light yellow oil. Compound 5: ¹H NMR (CDCl₃): δ 0.88 (t, J = 7.1 Hz, 3H), 1.18 (m, 1H), 1.33 (m, 3H), 2.04 (d, J = 0.7 Hz, 3H), 2.07 (m, 1H), 2.27 (m, 1H), 3.86 (d, J = 16.1 Hz, 1H), 3.90 (d, J = 16.1 Hz, 1H), 5.04 (dd, J = 9.0, 6.8 Hz, 1H), 6.96 (br. s, 1H), 6.98 (br. s, 1H), 7.23 (m, 3H), 7.31 (m, 2H).

Step 5: To a solution of 80 mg of compound **5** in 1 ml of N,N-dimethylformamide at room temperature and under a positive nitrogen atmosphere, was added 78 mg of (S)-compound 6, 0.057 ml of diisopropylethylamine, and 137 mg of HBTU. The mixture was stirred for 16 hours and then mixed with 1:1 ethyl acetate / hexane. This mixture was washed with 2N hydrochloric acid, saturated sodium bicarbonate, water (2X), and finally brine. The resulting solution was dried over magnesium sulfate and concentrated *in vacuo* to yield 156 mg of a yellow oil. This material was further purified by silica gel chromatography using 25% ethyl acetate as the eluant to give 109 mg of compound **7** as a colorless oil. Compound 7: (least polar diastereomer): ¹H NMR (CDCl₃): δ 0.85 (t, J = 7.1 Hz, 3H), 1.11 (t, J =7.1 Hz, 3H), 1.18 (m, 1H), 1.30 (m, 3H), 1.78 (m, 1H), 2.02 (d, J = 0.8 Hz, 3H), 2.14 (m, 1H), 2.57 (dd, J = 15.4, 7.1 Hz, 1H), 2.66 (dd, J = 15.4, 6.6 Hz, 1H), 3.86 (br. s, 2H), 3.95 (q, J = 7.1 Hz, 2H), 5.17 (m, 1H), 5.42 (t, J = 7.7 Hz, 1H), 5.93 (s, 2H), 6.72 (m, 2H), 6.74 (m, 1H), 6.90 (m, 1H), 7.11 (br, s, 1H), 7.23 (m, 3H), 7.30 (m, 2H), 7.37 (d, J = 7.7 Hz, 1H).

Step 6: A solution composed of 109 mg of compound **7**, 3 ml of tetrahydrofuran, 1 ml of 2N sodium hydroxide, and 2 ml of methanol was stirred at room temperature until hydrolysis was complete. The mixture was then diluted with water and extracted with diethyl ether. The aqueous layer was acidified with 2N hydrochloric acid and extracted (3X) with ethyl acetate. The combined extracts were dried with magnesium sulfate and concentrated *in vacuo* to yield 103 mg of compound **8**, a 1:1 diasteroisomeric mixture, as an off-white foam.

The diastereomeric mixture was separated by reverse-phase HPLC using a 30 -55% acetonitrile / water gradient to yield Compound 9 (*R*,*S*) and Compound **10** (*S*,*S*).

Compound **9** (most polar diastereomer): ¹H NMR (CD₃SOCD₃): δ 0.83 (t, J = 7.1 Hz, 3H), 1.13 (m, 2H), 1.26 (m, 2H), 1.76 (m, 1H), 1.96 (s overlapping m, 4H), 2.62 (dd, J = 15.8,6.6 Hz, 1H), 2.70 (dd, J = 15.8, 8.4 Hz, 1H), 3.69 (d, J = 14.8 Hz, 1H), 3.73 (d, J = 14.8 Hz, 1H), 5.09 (m, 1H), 5.47 (dd, J = 9.2, 6.6 Hz, 1H), 6.71 (dd, J = 8.0, 1.5 Hz, 1H), 6.77 (d, J = 8.0 Hz, 1H), 6.84 (d, J = 1.5 Hz, 1H), 7.00 (d, J = 1.8 Hz, 1H), 7.14-7.30 (m, 6H), 8.70 (d, J = 8.1 Hz, 1H).

Compound **10**: (least polar diastereomer) NMR (CD₃SOCD₃): δ 0.76 (t, J = 7.3 Hz, 3H), 1.01 (m, 2H), 1.20 (m, 2H), 1.98 (br. s, 3H), 2.60 (dd, J = 15.8, 7.0 Hz, 1H), 2.68 (dd, J =15.8, 7.7 Hz, 1H), 3.71 (d, J = 15.0 Hz, 1H), 3.76 (d, J = 15.0 Hz, 1H), 5.05 (ddd, J = 8.0, 7.7, 7.0 Hz, 1H), 5.51 (dd, J = 9.2, 6.6 Hz, 1H), 5.98 (s, 2H), 6.77 (dd, J = 8.0, 1.4 Hz, 1H), 6.83, (d, J = 8.0 Hz, 1H), 6.89 (d, J = 1.4 Hz, 1H), 7.02 (d, J = 2.2 Hz, 1H), 7.18 (m, 1H), 7.25 (m, 4H), 7.38 (m, 1H), 8.79 (d, J = 8.0 Hz, 1H), 12.08 (br. s; 1H).

### Example 2

Compound **12**, (3*S*)-3-((2*R*,*S*)-2-(3-benzyl-5-methyl-2-oxo-1(2*H*)-pyridinyl)hexanoylamino)-3-(2,3-dihydro-1-benzofuran-5-yl)propanoic acid, shown below, was synthesized according to the procedure of Example 1, 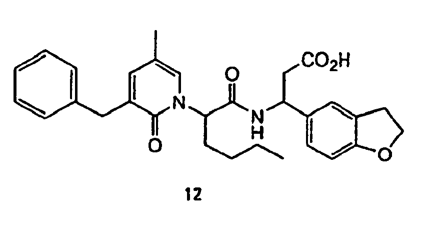 except that compound **A**, shown below, was substituted for compound **6** in step 5.

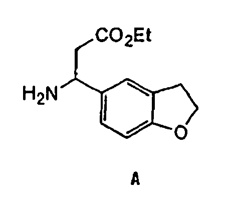

### Example 3

Compound **13**, (3*S*)-3-((2*R*,*S*)-2-(3-benzyl-5-methyl-2-oxo-1(2*H*)-pyridinyl)hexanoylamino)-3-(4-methylphenyl)propanoic acid, shown below, was obtained by the procedure of Example 1, 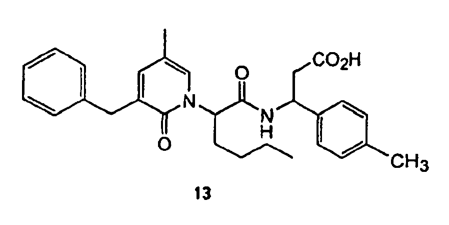 except that compound **B**, shown below, was substituted for compound **6** in step 5.

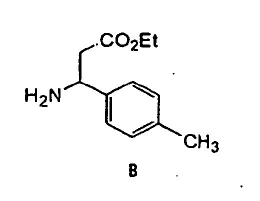

### Example 4

Compound **14,** (3*S*)-3-((2*R*,*S*)-2-(3-benzyl-5-methyl-2-oxo-1(2*H*)-pyridinyl)hexanoylamino)-3-(4-fluorophenyl)propanoic acid, shown below was obtained by the procedure of Example 1, 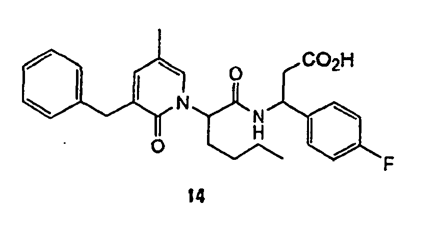 except that compound **11,** shown below, was substituted for compound **6** in step 5.

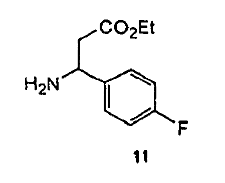

### Example 5

Compound **15**, (3*S*)-3-(1,3-benzodioxol-5-yl)-3-((2*R*,*S*)-2-(3-(4-methoxybenzyl)-5-methyl-2-oxo-1(2*H*)-pyridinyl)hexanoylamino)propanoic acid, shown below, can be obtained by the procedure of Example 1, 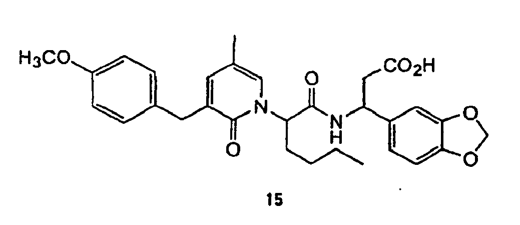 except that 3-(4-methoxyphenyl)-propanoyl chloride should be substituted for 3-phenylpropanoyl chloride in step 2.

### Example 6

Compound **16,** (3*S*)-3-(1,3-benzodioxol-5-yl)-3-((2*R*,*S*)-2-(3-(4-methylbenzyl)-5-methyl-2-oxo-1(2*H*)-pyridinyl)hexanoylamino)propanoic acid, shown below can be obtained by the procedure of Example 1, 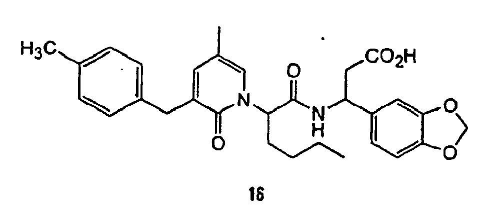 except that 3-(4-methylphenyl)-propanoyl chloride should be substituted for 3-phenylpropanoyl chloride in step 2.

### Example 7

Compound **17**, (3*S*)-3-(1,3-benzodioxol-5-yl)-3-((2*R*,*S*)-2-(3-(4-fluorobenzyl)-5-methyl-2-oxo-1(2*H*)-pyridinyl)hexanoylamino)propanoic acid, shown below, was obtained by the procedure of Example 1, 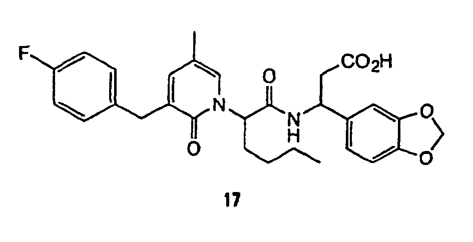 except that 3-(4-fluorophenyl)-propanoyl chloride was substituted for 3-phenylpropanoyl chloride in step 2.

### Example 8

Compound **18,** (3*S*)-3-(1,3-benzodioxol-5-yl)-3-((2*R*,*S*)-2-(3-(4-chlorobenzyl)-5-methyl-2-oxo-1(2*H*)-pyridinyl)hexanoylamino)propanoic acid, shown below was obtained by the procedure of Example 1, 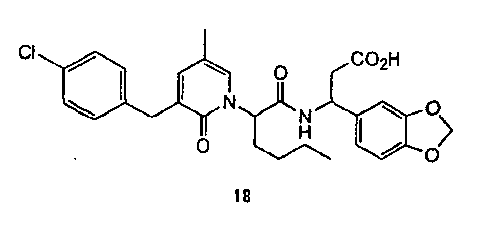 except that 3-(4-chlorophenyl)-propanoyl chloride was substituted for 3-phenylpropanoyl chloride in step 2.

### Example 9

Compound **19**, (3*S*)-3-(1,3-benzodioxol-5-yl)-3-((2*R*,*S*)-2-(3-(3-chlorobenzyl)-5-methyl-2-oxo-1(2*H*)-pyridinyl)hexanoylamino)propanoic acid, shown below was obtained by the procedure of Example 1, 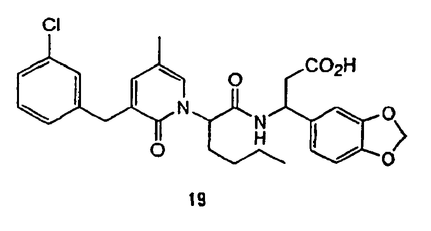 except that 3-(3-chlorophenyl)-propanoyl chloride was substituted for 3-phenylpropanoyl chloride in step 2.

### Example 11

### Synthesis of (3S)-3-(1,3-benzodioxol-5-yl)-3-({(2S)-2-[2-oxo-3-(phenylcarbonyl)-1(2H)-pyridinyl]hexanoyl}amino)propanoic acid (31).

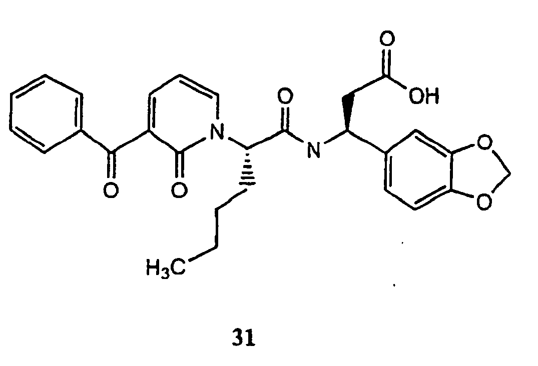

Step One: A solution of **23** (541 mg, 1.54 mmol) and ethyl benzoylacetate (0.53 mL, 3.09 mmol) in toluene (15 mL) was heated to reflux for 2 hours. The resulting mixture was cooled to room temperature and concentrated under reduced pressure. The residue was recrystallized from hexanes/CH₂Cl₂ to give compound **32** (310 mg, 40%) as a pale yellow solid.

Step Two: To a suspension of **32** (851 mg, 1.71 mmol) in ethanol (absolute, 6.8 mL) and acetic acid (glacial, 0.34 mL) at room temperature under nitrogen, 3-(dimethylamino)acrolein (1.02 mL, 10.2 mmol) was added by syringe. The resulting mixture was heated to reflux overnight, cooled to room temperature and diluted with ethyl acetate. This mixture was washed with HCl (2N, twice) and brine. The organic phase was dried over MgSO₄ and filtered and the filtrate was concentrated under reduced pressure. The residue was purified by silica gel chromatography, eluting with 3:2 hexanes:ethyl acetate to give **33** (476 mg, 52%) as a light yellow oil.

Step Three: To a solution of **33** (115 mg, 0.22 mmol) in THF (6 mL) at room temperature, aqueous NaOH (2N, 2 mL) and methanol (4 mL) were added. The resulting solution was stirred for 15 minutes, diluted with water and extracted with Et₂O. The aqueous phase was acidified with HCl (2N) and was extracted with ethyl acetate. The ethyl acetate layer was washed with water and brine, dried over MgSO₄ and filtered. The filtrate was concentrated under reduced pressure to give (3S)-3-(1,3-benzodioxol-5-yl)-3-({(2S)-2-[2-oxo-3-(phenylcarbonyl)-1(2H)-pyridinyl)hexanoyl}amino)propanoic acid (**31**, 100 mg, 92%) as a pale yellow foam. ¹H NMR (400 MHz, CD₃SO₂CD₃): δ 0.81 (t, J = 7.3 Hz, 3H), 1.08 (m, 2H), 1.25 (m, 2H), 1.80 (m, 1H), 1.93 (m, 1H), 2.61 (dd, J = 15.8, 6.8 Hz, 1H), 2.68 (dd, J = 15.8, 7.9 Hz, 1H), 5.09 (m, 1H), 5.49 (dd, J = 9.5, 6.2 Hz, 1H), 5.98 (s, 2H), 6.24 (t, J = 7.0 Hz, 1H), 6.78 (dd, J = 8.1, 1.4 Hz, 1H), 6.84 (d, J = 8.1 Hz, 1H), 6.89 (d, J = 1.4 Hz, 1H), 7.49 (t, J = 7.7 Hz, 2H), 7.62 (m, 1H), 7.70 (m, 3H), 7.97 (dd, J = 7.0, 2.2 Hz, 1H), 8.87 (d, J = 8.1 Hz, 1H), 12.11 (br. s, 1H).

### Example 12

### Synthesis of (3S)-3-(1,3-benzodioxol-5-yl)-3-({(2S)-2-[2-oxo-3-(phenylmethyl)-1(2H)-pyridinyl]hexanoyl}amino)propanoic acid (34).

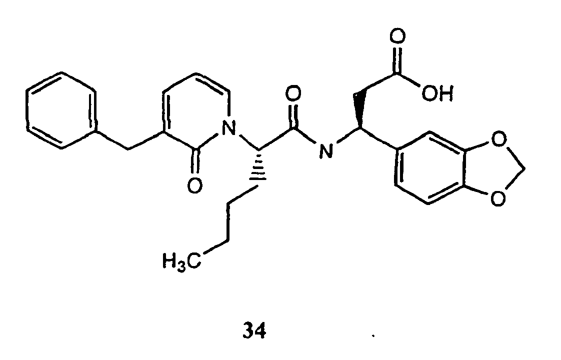

Step One: To a solution of **33** (88 mg, 0.17 mmol) in ethanol (absolute, 4 mL) at room temperature, NaBH₄ (12.5 mg, 0.33 mmol) was added. The resulting mixture was stirred for 20 minutes, then was quenched with HCl (2N, 2 mL). The resulting mixture was diluted with water and ethyl acetate and the organic layer was washed with saturated aqueous NaHCO₃ and brine. The organic layer was dried over MgSO₄ and filtered and the filtrate was concentrated under reduced pressure to give **35** (85 mg, 96%) as a pale yellow oil. This material was used without purification.

Step Two: To a solution of **35** (85 mg, 0.16 mmol) in ethyl acetate (4 mL) at room temperature under nitrogen, Pd/C (10% dry weight basis, Degussa type E101 NE/W, ~50% water content, 36 mmol) was added. The atmosphere was replaced with hydrogen (toggle between vacuum and hydrogen from a balloon five times) and the mixture was vigorously stirred for 1.5 hours. The mixture was filtered through Celite and the filtrate was concentrated under reduced pressure. The residue was purified by silica gel chromatography, eluting with 7:3 hexanes:ethyl acetate to give **36** (32 mg, 39%) as a colorless oil.

Step Three: To a solution of **36** (32 mg, 0.062 mmol) in THF (3 mL) at room temperature, aqueous NaOH (2N, 1 mL) and methanol (2 mL) were added. The resulting solution was stirred for 15 minutes, diluted with water and extracted with Et₂O. The aqueous phase was acidified with HCl (2N) and was extracted with ethyl acetate. The ethyl acetate layer was washed with water and brine, dried over MgSO₄ and filtered. The filtrate was concentrated under reduced pressure. The residue was taken up in acetonitrile (3 mL) and water (7 mL) and the mixture was lyophilized to give (3S)-3-(1,3-benzodioxol-5-yl)-3-({(2S)-2-[2-oxo-3-(phenylmethyl)-1(2H)-pyridinyl]hexanoyl}amino)propanoic acid (**34**, 31 mg, 100%) as a white powder. ¹H NMR (400 MHz, CD₃SO₂CD₃): δ 0.76 (t, J = 7.3 Hz, 3H), 1.01 (m, 2H), 1.22 (m, 2H), 1.70 (m, 1H), 1.87 (m, 1H), 2.60 (dd, J = 15.8, 7.0 Hz, 1H), 2.68 (dd, J =15.8, 7.9 Hz, 1H), 3.72 (d, J = 15.0 Hz, 1H), 3.77 (d, J = 15.0 Hz, 1H), 5.06 (m, 1H), 5.54 (dd, J = 9.2, 6.6 Hz, 1H), 5.98 (s, 2H), 6.16 (t, J = 7.0 Hz, 1H), 6.77 (dd, J = 8.1, 1.4 Hz, 1H), 6.83 (d, J = 8.1 Hz, 1H), 6.89 (d, J = 1.4 Hz, 1H), 7.13 (m, 1H), 7.18 (m, 1H), 7.26 (m, 4H), 7.59 (dd, J = 7.0, 1.8 Hz, 1H), 8.83 (d, J = 8.1 Hz, 1H), 12.11 (br. s, 1H).

### Example 13

### Synthesis of (3S)-3-(1,3-benzodioxol-5-yl)-3-[({1-[2-oxo-3-(phenylmethyl)-1(2H)-pyridinyl]cyclohexyl}carbonyl)amino]propanoic acid.

Step One: To a solution of 3-benzylpyridine (1.65 g, 9.77 mmol) in acetone (3.5 mL), 1-chloro-2,4-dinitrobenzene (2.00g, 9.56 mmol) was added and the mixture was refluxed overnight. The mixture was cooled to room temperature, diluted with acetone and the solvent was decanted from the precipitate. The crude solid was washed with acetone (2 times) and diethyl ether (1 time), decanting each time to give **37** (3.57 g, 100%) as a gray solid.

Step Two: To a solution of 1-amino-1-hydroxymethylcyclohexane (0.45g, 3.5 mmol) in n-butanol (8.75 mL), solid N-(2,4-dintrophenyl)-3-benzylpyridinum chloride (37, 1.23 g, 3.3 mmol) was added. The resulting solution was heated to reflux for 2.5 days under a nitrogen atmosphere. The mixture was cooled, diluted with water and filtered. The filtrate was basified with concentrated NH₄OH (2mL) and extracted with ethyl acetate. The aqueous layer was concentrated to dryness to give **38** (0.56 g) as a yellow oil which was used without further purification.

Step Three: To a solution of crude **38** (0.56g, 3.5 mmol theoretical) in water (10 mL), a solution of potassium ferricyanide (3.3 g, 10 mmol) in water (15 mL) was added dropwise via an addition funnel over 30 minutes at 0 °C. A solution of KOH (0.76 g, 13.5 mmol) in water (5 mL) was then added over 30 minutes. Toluene (10 mL) was added and the solution was stirred for one hour at 0 °C. The layers were separated, and the aqueous layer was extracted again with toluene. The combined extracts were dried over Na₂SO₄ and filtered and the filtrate was concentrated under reduced pressure. The residue was chromatographed on silica gel, eluting with 7:13 hexanes:ethyl acetate to give **39** (20 mg, 1.9%, two steps.)

Step Four: To a suspension of **39** (20 mg, 0.068 mmol) in aqueous KOH (1M, 0.70 mL) potassium persulfate (0.073 g, 0.270 mmol) and ruthenium (III) chloride (1mg, catalytic) and THF (0.25 mL) were added. The mixture was stirred for 1 hour and extracted with dichloromethane. The aqueous layer was acidified and extracted with ethyl acetate (3 times). The ethyl acetate extracts were combined, dried over MgSO₄ and filtered. The filtrate was concentrated under reduced pressure to give **40** (0.0148 g, 70%) as a tan solid.

(3S)-3-(1,3-Benzodioxol-5-yl)-3-[({1-[2-oxo-3-(phenylmethyl)-1(2H)-pyridinyl]cyclohexyl}carbonyl)amino]propanoic acid was prepared from **40** according to the procedures described in Example 1. ¹H NMR (400 MHz, CD₃SO₂CD₃): δ 1.40 (m, 4H), 1.68 (m, 2H), 2.04 (m, 2H), 2.60 (d, J = 7.0 Hz, 2H), 3.67 (d, J = 15.2 Hz, 1H), 3.72 (d, J = 15.2 Hz, 1H), 5.12 (m, 1H), 5.95 (m, 2H), 6.19 (t, J = 7.0 Hz, 1H), 6.74 (dd, J = 7.8, 1.4 Hz, 1H), 6.76 (d, J = 7.8 Hz, 1H), 6.90 (d, J = 1.4 Hz, 1H), 7.10 (d, J = 5.8 Hz, 1H), 7.20 (m, 5H), 7.57 (d, J = 8.4 Hz, 1H), 7.66 (dd, J = 7.7, 1.8 Hz, 1H).

### Example 14

### Synthesis of (3S)-3-(1,3-benzodioxol-5-yl)-3-({2-[2-oxo-5-(phenylmethyl)-1(2H)-pyridinyl]hexanoyl}amino)propanoic acid.

Step One: To a mixture of **41** (prepared according to procedures described in Example 13, 1.75 g crude orange oil, 5.0 mmol theoretical) in water (25 mL) at 0 °C, a solution of potassium ferricyanide (4.7 g, 14 mmol) in water (22 mL) was added dropwise *via* an addition funnel over 30 minutes. A solution of KOH (1.1 g, 19 mmol) in water (7 mL) was then added over 30 minutes. Toluene (15 mL) was added and the solution was stirred for one hour at 0 °C. The layers were separated, and the aqueous layer was extracted again with toluene. The combined extracts were dried over Na₂SO₄ and filtered and the filtrate was concentrated under reduced pressure. The residue was chromatographed on silica gel, eluting with 7:13 hexanes:ethyl acetate to give **42** (major product, 0.36 g, 29%) and **43** (minor product, 0.10 g, 7.0%).

(3S)-3-(1,3-Benzodioxol-5-yl)-3-({2-[2-oxo-5-(phenylmethyl)-1(2H)-pyridinyl]hexanoyl}amino)propanoic acid was prepared from **42** according to procedures described in Examples 1 and 13. ¹H NMR (400 MHz, CD₃SO₂CD₃) δ: 0.77 (t, J = 7.3 Hz, 3H), 1.00 (m, 2H), 1.20 (m, 2H), 1.75 (m, 1H), 1.88 (m, 1H), 2.65 (m, 2H), 3.70 (s, 2H), 5.08 (m, 1H), 5.49 (dd, J = 9.9, 6.2 Hz, 1H), 5.98 (s, 2H), 6.32 (d, J = 9.2 Hz, 1H), 6.77 (dd, J = 8.1, 1.5 Hz, 1H), 6.83 (d, J = 9.2 Hz, 1H), 6.89 (d, J = 1.5 Hz, 1H), 7.20 (m, 4H), 7.28 (m, 4H), 7.61 (d, J = 2.6 Hz, 1H), 8.81 (d, J = 8.1 Hz, 1H), 12.10 (br. s, 1H).

### Example 22

The ability of compounds of the present invention to inhibit binding is determined by a procedure in which a 26-amino acid peptide containing the CS 1 sequence of fibronectin with an N-terminal Cys (CDELPQLVTLPHPNLHGPEILDVPST) was coupled to maleimide activated ovalbumin. Bovine serum albumin (BSA) and CS 1 conjugated ovalbumin were coated onto 96-well polystyrene plates at 0.5 µg/ml in TBS (50 mM Tris, pH 7.5; 150 mM NaCl) at 4°C for 16 hours. The plates were washed three times with TBS and blocked with TBS containing 3% BSA at room temperature for 4 hours. Blocked plates were washed three times in binding buffer (TBS; 1 mM MgCl₂; 1 mM CaCl₂; 1 mM MnCl₂) prior to assay. Ramos cells fluorescently labeled with calcein AM were resuspended in binding buffer (10⁷ cells/ml) and diluted 1:2 with same buffer with or without compound. The cells were added immediately to the wells (2.5 x 10⁵ cells/well) and incubated for 30 minutes at 37°C. Following three washes with binding buffer, adherent cells were lysed and quantitated using a fluorometer. The results are shown in Tables 1, 2 and 3. IC₅₀ is defined as the dose required to give 50% inhibition. MS in Table 3 stands for Mass Spec. nd stands for not determined in the Tables. A stands for inhibition in Table 2, and the percent inhibition indicates the inhibition of cell adhesion when compound is included in the assay at a concentration of 100 µM. The lower the IC₅₀ value and the greater the percentage of inhibition, the more efficient the compound is at prevention of cell adhesion.

**Table 1**

| Compound Number | IC₅₀ (nM) | Mass Spectral Data (m/z) |
|---|---|---|
| 8 | 7 | Calc'd (M-H)⁻ = 503.22 Found (M-H)- = 503.24 |
| 9 | 2000 | Calc'd (M-H)- = 503.22 Found (M-H)⁻ = 503.24 |
| 12 | 40 | Calc'd (M-H)- = 501.24 Found (M-H)⁻ = 501.27 |
| 13 | 70 | Calc'd (M-H)⁻ = 473.24 Found (M-H)⁻ = 473.26 |
| 14 | 150 | Calc'd (M-H)⁻ = 477.22 Found (M-H)- = 477.25 |

**Table 3**

| Compound | Mass Spectral Data (m/z) | IC₅₀ (µM) |
|---|---|---|
| (3S)-3-(1,3-benzodioxol-5-yl)-3-({2-[2-oxo-3-[(2-thiophenylmethyl)amino]-1(2H)-pyridinyl]hexanoyl}amino)propanoic acid | Calc'd(M-H)⁻ = 510.17 Found (M-H)⁻ = 510.21 | 1.3 |
| (3S)-3-(1,3-benzodioxol-5-yl)-3-({2-[2-oxo-3-(phenylmethyl)-1(2H)-pyridinyl]butanoyl}amino)propanoic acid | Calc'd (M-H)⁻=461.17 Found (M-H)⁻ = 461.18 | 0.03 |
| (3S)-3-(1,3-benzodioxol-5-yl)-3-({(2S)-2-[2-oxo-3-(phenylcarbonyl)-1 (2H)-pyridinyl]hexanoyl}amino)propanoic acid, 31 | Calc'd (M-H)⁻ = 503.18 Found (M-H)⁻ = 503.18 | 0.55 |
| (3S)-3-(1,3-benzodioxol-5-yl)-3-({2-[2-oxo-3-(phenylmethyl)-1(2H)-pyridinyl]acetyl}amino)propanoic acid | Calc'd (M-H)⁻ = 433.14 Found (M-H)⁻ = 433.16 | 0.45 |
| (3S)-3-(1,3-benzodioxol-5-yl)-3-[({1-[2-oxo-3-(phenylmethyl)-1(2H)-pyridinyl]cyclohexyl}carbonyl)amino]propanoic acid | Calc'd (M-H)⁻ = 501.20 Found (M-H)⁻ = 501.24 | 50 |
| (3S)-3-(1,3-benzodioxol-5-yl)-3-({(2S)-2-[2-oxo-3-(phenyhnethyl)-1(2H)-pyridinyl]hexanoyl}amino)propanoic acid, 34 | Calc'd (M-H)⁻ = 489.20 Found (M-H)⁻ = 489.20 | 0.004 |
| (3S)-3-(1,3-benzodioxol-5-yl)-3-({2-[3-[(2-chlorophenyl)methyl]-5-methyl-2-oxo-1(2R)-pyridinyl]hexanoyl}amino)propanoic acid | Calc'd (M-H)⁻ = 537.18 Found (M-H)⁻ = 537.22 | 0.01 |
| (3S)-3-(1,3-benzodioxol-5-yl)-3-({2-[2-oxo-5-(phenylmethyl)-1(2H)-pyridinyl]hexanoyl}amino)propanoic acid | Calc'd (M-H)⁻ = 489,20 Found (M-H)⁻ = 489.21 | 0.04 |
| (3S)-3-(1,3-benzodioxol-5-yl)-3-({2-[2-oxo-3-(phenylmethyl)-1(2H)-pyridinyl)pentanoyl}amino)propanoic acid | Calc'd (M-H)⁻ = 475.18 Found (M-H)⁻ = 475.19 | 0.06 |

The present invention is illustrated by way of the foregoing description and examples. The foregoing description is intended as a non-limiting illustration, since many variations will become apparent to those skilled in the art in view thereof.

Changes can be made in the composition, operation and arrangement of the method of the present invention described herein without departing from the concept and scope of the invention as defined in the following claims:

### SEQUENCE LISTING

(1) GENERAL INFORMATION:
   (i) APPLICANT: Biediger, Ronald J.; Holland, George W.; Kassir, Jamal M.; Market, Robert V.; Li, Wen; Scott, Ian L. and Wu, Chengde
   (ii) TITLE OF INVENTION: Propanoic Acid Derivatives that Inhibit the Binding of Integrins to their Receptors
   (iii) NUMBER OF SEQUENCES: 1
   (iv) CORRESPONDENCE ADDRESS:
      (A) ADDRESSEE: Rockey, Milnamow & Katz, Ltd.
      (B) STREET: 180 N. Stetson Avenue, 2 Prudential Plaza, Suite 4700
      (C) CITY: Chicago
      (D) STATE: IL
      (E) COUNTRY: U.S.A.
      (F) ZIP: 60601
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Katz, Martin L.
      (B) REGISTRATION NUMBER: 25,011
      (C) REFERENCE/DOCKET NUMBER: TEX4542P0410US
   (ix) TELECOMMUNICATION INFORMATION:
      (A) TELEPHONE: 312-616-5400
      (B) TELEFAX: 312-616-5460
(2) INFORMATION FOR SEQ ID NO: 1:
   (i) SEQUENCE CHARACTERISTICS:
      (A) LENGTH: 26 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 1:
   Cys Asp Glu Leu Pro Gln Leu Val Thr Leu Pro His Pro Asn Leu His
   1               5                   10                  15
   Gly Pro Glu Ile Leu Asp Val Pro Ser Thr
               20                  25

## Claims

1. A compound of the structure 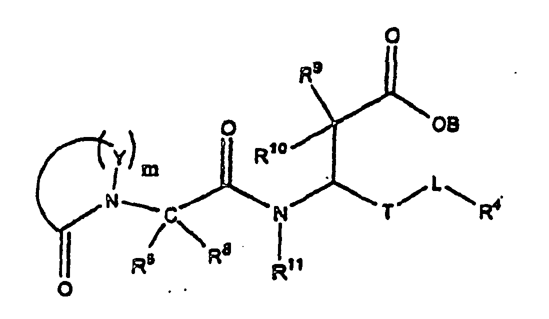 wherein the ring including Y is a mono-cyclic heterocycle consisting of an optionally substituted oxo-pyridinyl of the formula IV: 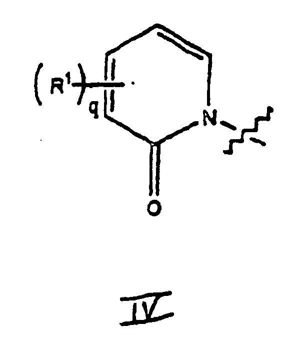
q is an integer of zero to four; and
T is selected from the group consisting of (CH₂)_{b} wherein b is an integer of 0 to 3;
L is selected from the group consisting of O, NR¹³, S, and (CH₂)ₙ wherein n is an integer of 0 or 1; and
B, R¹, R⁴, R⁶, R⁹ , R¹⁰, R¹¹ and R¹³ are independently selected from the group consisting of hydrogen, halogen, hydroxyl, alkyl alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), -NHC(O)NH(C₁-C₃ alkyl), -NHC(O)N(C₁-C₃ alkyl)C(O)NH(C₁-C₃ alkyl), -C₁-C₃ alkylamino, alkenylamino, alkynylamino, di(C₁-C₃ alkyl)amino, -C(O)O-(C₁-C₃ alkyl), -C(O)NH-(C₁-C₃ alkyl), - CH=NOH, -PO₃H₂, -OPO₃H₂, -C(O)N(C₁-C₃ alkyl)₂, haloalkyl, alkoxyalkoxy, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, aryloxyalkyl, carboxyl, carbamate and - C(O)NH(benzyl);
R⁸, is independently selected from the group consisting of halogen, hydroxyl, alkyl, alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), -NHC(O)NH(C₁-C₃ alkyl), -NHC(O)N(C₁-C₃ alkyl)C(O)NH(C₁-C₃ alkyl), -C₁-C₃ alkylamino, alkenylamino, alkynylamino, di(C₁-C₃ alkyl)amino, -C(O)O-(C₁-C₃ alkyl), -C(O)NH-(C₁-C₃ alkyl), - CH=NOH, -PO₃H₂, -OPO₃H₂, -C(O)N(C₁-C₃ alkyl)₂, haloalkyl alkoxyalkoxy, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, aryloxyalkyl, carboxyl, carbamate and - C(O)NH(beazyl);
wherein when L is -NR¹³-, R⁴ and R¹³ taken together may form a ring;
and wherein R⁶ and R⁸ taken together may form a ring; and wherein R⁹ and R¹⁰ taken together may form a ring;
or a pharmaceutically acceptable salt thereof.

2. The compound of claim 1 which is a derivative thereof selected from esters, carbamates and aminals.

3. A compound of the structure 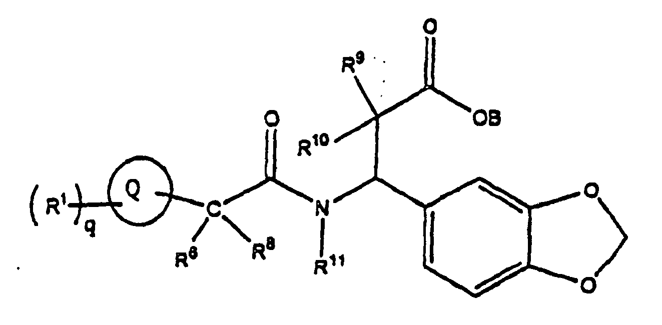 wherein circle Q is a ring consisting of 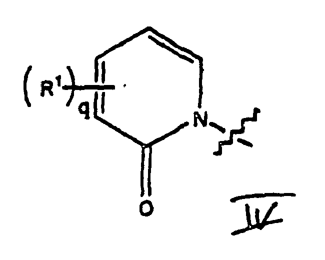 q is an integer of zero to four, and,
B, R¹, R⁶, R⁸, R⁹, R¹⁰ and R¹¹ are each independently selected from the group consisting of hydrogen, halogen, hydroxyl, alkyl, alkenyl, alkynyl, alkoxy, alkenoxy, alkynoxy, thioalkoxy, hydroxyalkyl, aliphatic acyl, -CF₃, nitro, amino, cyano, carboxy, -N(C₁-C₃ alkyl)-C(O)(C₁-C₃ alkyl), -NHC(O)NH(C₁-C₃ alkyl), -NHC(O)N(C₁-C₃ alkyl)C(O)NH(C₁-C₃ alkyl), -C₁-C₃ alkylamino, alkenylamino, alkynylamino, di(C₁-C₃ alkyl)amino, -C(O)O-(C₁-C₃ alkyl), -C(O)NH-(C₁-C₃ alkyl), -CH=NOH, -PO₃H₂, -OPO₃H₂, -C(O)N(C₁-C₃ alkyl)₂, haloalkyl, alkoxyalkoxy, carboxaldehyde, carboxamide, cycloalkyl, cycloalkenyl, cycloalkynyl, cycloalkylallcyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, sulfonyl, -SO₂-(C₁-C₃ alkyl), -SO₃-(C₁-C₃ alkyl), sulfonamido, aryloxyalkyl, carboxyl, carbamate and -C(O)NH(benzyl);
R⁶ and R⁸ taken together may form a ring;
and wherein R⁹ and R¹⁰ taken together may form a ring;
or a pharmaceutically acceptable salt thereof.

4. The compound of claim 3 which is a derivative thereof selected from esters, carbamates and animals.

5. The compound of claim 3 wherein R⁶, R⁸, R⁹ , R¹⁰ and R¹¹ are each independently selected from the group consisting of hydrogen and alkyl, and R¹
at each occurrence, is independently selected from the group consisting of hydrogen, 2-thienylmethyl, benzyl and methyl.

6. A compound selected from the group consisting of
(3*S*)-3-(1,3-benzodioxol-5-yl)-3-((2*R*,*S*)-2-(3-benzyl-5-methyl-2-oxo-1(2*H*)-pyridinyl)hexanoylamino)propanoic acid, (3*S*)-3-(1,3-benzodioxol-5-yl)-3-((2*R*,*S*)-2-(3-(3-chlorobenzyl)-5-methyl-2-oxo-1(2*H*)-pyridinyl)hexanoylamino)propanoic acid, (3S)-3-(1,3-benzodioxol-5-yl)-3-(((2*S*)-2-(2-oxo-3-(phenylmethyl)-1(2H)-pyridinyl)hexanoyl)amino) propanoic acid, (3*S*)-3-(1,3-benzodioxol-5-yl)-3-((2-(3-cholorophenyl)methyl)-5-methyl-2-oxo-1(2H)-pyridinyl)hexanoyl)amino)propanoic acid and pharmaceutically acceptable salts thereof.

7. The compound of claim 6 which is a derivative thereof selected from esters, carbamates and aminals.

8. A pharmaceutical composition comprising:
a compound of claim 1;
in a pharmaceutically acceptable carrier.

9. Use of a compound of claim 1 for preparing a drug for selectively inhibiting α₄β₁ integrin binding in a mammal.

## Patentansprüche

1. Eine Verbindung mit der Struktur 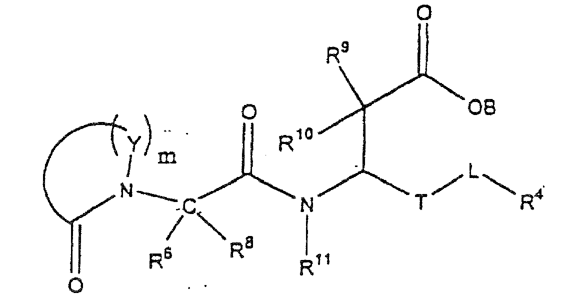 worin der Ring einschließlich Y ein monozyklischer Heterozyklus ist, bestehend aus einem wahlweise substituierten oxo-Pyridinyl der Formel IV: 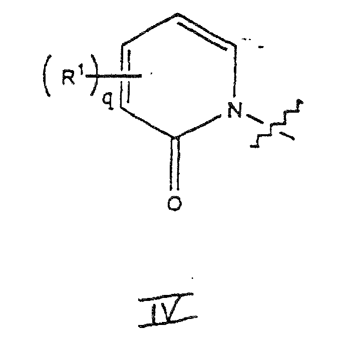
q ist eine ganze Zahl von null bis vier; und
T ist gewählt aus der Gruppe bestehend aus (CH₂)_{b}, worin b eine ganze Zahl von 0 bis 3 ist;
L ist gewählt aus der Gruppe bestehend aus 0, NR¹³, S und (CH₂)ₙ, worin n eine ganze Zahl von 0 oder 1 ist; und
B, R¹, R⁴, R⁶, R⁹, R¹⁰, R¹¹ und R¹³ sind unabhängig gewählt aus der Gruppe bestehend aus Wasserstoff, Halogen, Hydroxyl, Alkyl, Alkenyl, Alkinyl, Alkoxy, Alkenoxy, Alkinoxy, Thioalkoxy, Hydroxyalkyl, aliphatisches Acyl, -CF₃, Nitro, Amino, Cyano, Carboxy, -N(C₁-C₃ Alkyl) -C (O) (C₁-C₃ Alkyl), -NHC(O)NH(C₁-C₃ Alkyl), -NHC(O)N(C₁-C₃ Alkyl)C(O)NH(C₁-C₃ Alkyl), Alkenylamino, Alkinylamino, di(C₁-C₃ Alkyl)amino, -C(O)O-(C₁-C₃ Alkyl), -C(O)NH-(C₁-C₃ Alkyl), -CH=NOH, -PO₃H₂, -OPO₃H₂, -C(O)N(C₁-C₃ Alkyl)₂, Haloalkyl, Alkoxyalkoxy, Carboxaldehyd, Carboxamid, Cycloalkyl, Cycloalkenyl, Cycloalkinyl, Cycloalkylalkyl, Aryl, Aroyl, Aryloxy, Arylamino, Biaryl, Thioaryl, Diarylamino, Heterocyclyl, Alkylaryl, Aralkenyl, Aralkyl, Alkylheterocyclyl, Heterocyclylalkyl, Sulfonyl, -SO₂- (C₁-C₃ Alkyl), -SO₃-(C₁-C₃ Alkyl), Sulfonamido, Aryloxyalkyl, Carboxyl, Carbamat und - C(O)NH(Benzyl);
R⁸ ist unabhängig gewählt aus der Gruppe bestehend aus Halogen, Hydroxyl, Alkyl, Alkenyl, Alkinyl, Alkoxy, Alkenoxy, Alkinoxy, Thioalkoxy, Hydroxyalkyl, aliphatisches Acyl, -CF₃, Nitro, Amino, Cyano, Carboxy, -N (C₁-C₃ Alkyl)-C(O)(C₁-C₃ Alkyl), -NHC(O)NH(C₁-C₃ Alkyl), -NHC(O)N(C₁-C₃ Alkyl) C(O)NH(C₁-C₃ Alkyl), -C₁-C₃ Alkylamino, Alkenylamino, Alkinylamino, di(C₁-C₃ Alkyl)amino,-C(O)O-(C₁-C₃ Alkyl), -C(O)NH-(C₁-C₃ Alkyl), -CH=NOH, -PO₃H₂, - OPO₃H₂, -C(O)N(C₁-C₃ Alkyl)₂, Haloalkyl, Alkoxyalkoxy, Carboxaldehyd, Carboxamid, Cycloalkyl, Cycloalkenyl, Cycloalkinyl, Cycloalkylalkyl, Aryl, Aroyl, Aryloxy, Arylamino, Biaryl, Thioaryl, Diarylamino, Heterocyclyl, Alkylaryl, Aralkenyl, Aralkyl, Alkylheterocyclyl, Heterocyclylalkyl, Sulfonyl, -SO₂-(C₁-C₃ Alkyl), -SO₃-(C₁-C₃ Alkyl), Sulfonamido, Aryloxyalkyl, Carboxyl, Carbamat und -C(O)NH(Benzyl);
worin, wenn L -NR¹³- ist, R⁴ und R¹³ zusammengenommen einen Ring bilden können;
und worin R⁶ und R⁸ zusammengenommen einen Ring bilden können;
und worin R⁹ und R¹⁰ zusammengenommen einen Ring bilden können;
oder ein pharmazeutisch verträgliches Salz davon.

2. Die Verbindung von Anspruch 1, die ein Derivat davon ist, gewählt aus Estern, Carbamaten und Aminalen.

3. Eine Verbindung mit der Struktur 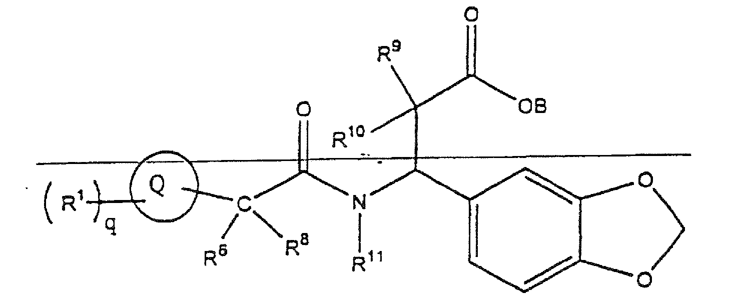 worin der Kreis Q ein Ring ist, bestehend aus 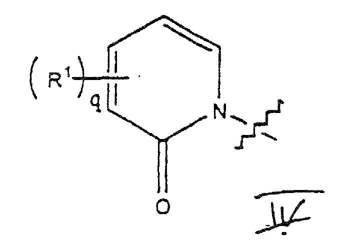
q ist eine ganze Zahl von null bis vier; und
B, R¹, R⁶, R⁸, R⁹, R¹⁰ und R¹¹ sind jeweils unabhängig gewählt aus der Gruppe bestehend aus Wasserstoff, Halogen, Hydroxyl, Alkyl, Alkenyl, Alkinyl, Alkoxy, Alkenoxy, Alkinoxy, Thioalkoxy, Hydroxyalkyl, aliphatisches Acyl, -CF₃, Nitro, Amino, Cyano, Carboxy, -N (C₁-C₃ Alkyl)-C(O) (C₁-C₃ Alkyl), -NHC(O)NH(C₁-C₃ Alkyl), -NHC (O)N (C₁-C₃ Alkyl)C(O)NH(C₁-C₃ Alkyl), -C₁-C₃ Alkylamino, Alkenylamino, Alkinylamino, di(C₁-C₃ Alkyl) amino, -C(O)O-(C₁-C₃ Alkyl), -C(O)NH-(C₁-C₃ Alkyl), -CH=NOH, -PO₃H₂, -OPO₃H₂, -C(O)N(C₁-C₃ Akyl)₂, Haloalkyl, Alkoxyalkoxy, Carboxaldehyd, Carboxamid, Cycloalkyl, Cycloalkenyl, Cycloalkinyl, Cycloalkylalkyl, Aryl, Aroyl, Aryloxy, Arylamino, Biaryl, Thioaryl, Diarylamino, Heterocyclyl, Alkylaryl, Aralkenyl, Aralkyl, Alkylheterocyclyl, Heterocyclylalkyl, Sulfonyl, -SO₂-(C₁-C₃ Alkyl), -SO₃-(C₁-C₃ Alkyl), Sulfonamido, Aryloxyalkyl, Carboxyl, Carbamat und - C(O)NH(Benzyl);
worin R⁶ und R⁸ zusammengenommen einen Ring bilden können;
und worin R⁹ und R¹⁰ zusammengenommen einen Ring bilden können;
oder ein pharmazeutisch verträgliches Salz davon.

4. Die Verbindung von Anspruch 3, die ein Derivat davon ist, gewählt aus Estern, Carbamaten und Aminalen.

5. Die Verbindung von Anspruch 3, worin R⁶, R⁸, R⁹, R¹⁰ und R¹¹ jeweils unabhängig gewählt sind aus der Gruppe bestehend aus Wasserstoff und Alkyl, und R¹ ist bei jedem Auftreten unabhängig gewählt aus der Gruppe bestehend aus Wasserstoff, 2-Thienylmethyl, Benzyl und Methyl.

6. Eine Verbindung gewählt aus der Gruppe bestehend aus
(3*S*)-3-(1,3-Benzodioxol-5-yl)-3-((2*R*,*S*)-2-(3-benzyl-5-methyl-2-oxo-1(2*H*)-pyridinyl)hexanoylamino)propansäure, (3S)-3-(1,3-Benzodioxol-5-yl)-3-((2*R*,*S*)-2-(3-(3-chlorbenzyl)-5-methyl-2-oxo-1(2*H*)-pyridinyl)hexanoylamino)propansäure, (3S)-3-(1,3-Benzodioxol-5-yl)-3-(((2*S*)-2-(2-oxo-3-(phenylmethyl)-1(2H)-pyridinyl)hexanoyl)amino)propansäure, (3S)-3-(1,3-Benzodioxol-5-yl)-3-((2-(3-chlorphenyl)methyl)-5-methyl-2-oxo-1(2H)-pyridinyl)hexanoyl)amino)propansäure und
pharmazeutisch verträgliche Salze davon.

7. Die Verbindung von Anspruch 6, die ein Derivat davon ist, gewählt aus Estern, Carbamaten und Aminalen.

8. Eine pharmazeutische Zusammensetzung, die folgendes umfasst:
eine Verbindung von Anspruch 1;
in einem pharmazeutisch verträglichen Träger.

9. Verwendung einer Verbindung von Anspruch 1 zur Herstellung eines Arzneimittels zur selektiven Hemmung der α₄β₁ Integrinbindung in einem Säugetier.

## Revendications

1. Composé de structure 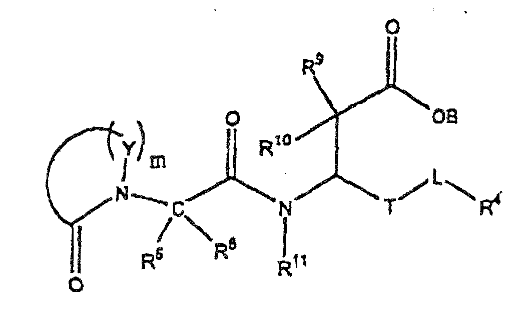 dans laquelle le cycle comprenant Y est un hétérocycle monocyclique comprenant un oxo-pyridinyle éventuellement substitué de formule IV : 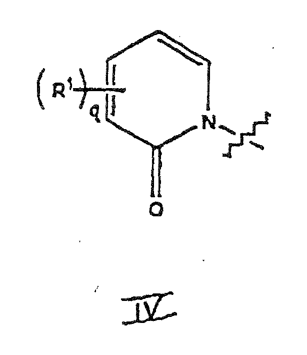
q est un nombre entier de zéro à quatre ; et
T est choisi dans le groupe constitué de (CH₂)_{b}, dans lequel b est un nombre entier de 0 à 3 ;
L est choisi dans le groupe constitué de O, NR¹³, S, et (CH₂)ₐ, où n est un nombre entier de 0 ou 1 ; et
B, R¹, R⁴, R⁶, R⁹, R¹⁰, R¹¹ et R¹³ sont choisis indépendamment dans le groupe constitué de l'hydrogène, de l'halogène, des groupes hydroxyle, alkyle, alkényle, alkynyle, alcoxy, alkénoxy, alkynoxy, thioalcoxy, hydroxyalkyle, acyle aliphatique, -CF₃, nitro, amino, cyano, carboxy, -N(alkyle en C₁-C₃) -C(O) (alkyle en C₁-C₃), -NHC(O)NH(alkyle en C₁-C₃),-NHC(O)N(alkyle en C₁-C₃)C(O)NH(alkyle en C₁-C₃),
alkénylamino, alkylylamino, di (alkyle en C₁-C₃)amino,-C(O)O-(alkyle en C₁-C₃), -C(O)NH-(alkyle en C₁-C₃), -CH=NOH,-PO₃H₂, -OPO₃HC(O)N(alkyle en C₁-C₃)₂, haloalkyle, alcoxyalcoxy, carboxaldéhyde, carboxamide, cycloalkyle, cycloalkényle, cycloalkynyle, cycloalkylalkyle, aryle, aroyle, aryloxy, arylamino, biaryle, thioaryle, diarylamino, hétérocyclyle, alkylaryle, aralkényle, aralkyle, alkylhétérocyclyle, hétérocyclylalkyle, sulfonyle, -SO₂-(alkyle en C₁-C₃), -SO₃ (alkyle en C₁-C₃), sulfonamido, aryloxyalkyle, carboxyle, carbamate, et -C(O)NH(benzyle) ;
R⁸ est choisi indépendamment dans le groupe constitué d'halogène, des groupes hydroxyle, alkyle, alkényle, alkynyle, alcoxy, alkénoxy, alkynoxy, thioalcoxy, hydroxyalkyle, acyle aliphatique, -CF₃, nitro, amino, cyano, carboxy, -N(alkyle en C₁-C₃)-C(O) (alkyle en C₁-C₃), -NHC(O)NH(alkyle en C₁-C₃),-NHC(O)N(alkyle en C₁-C₃) C (O)NH (alkyle en C₁-C₃), alkylamino en C₁-C₃, alkénylamino, alkynylamino, di(alkyle en C₁-C₃)amino, - C(O)O-(alkyle en C₁-C₃), -C(O)NH-(alkyle en C₁-C₃), -CH=NOH,-PO₃H₂, -OPO₃H₂, -C(O)N(alkyle en C₁-C₃)₂, haloalkyle, alcoxyalcoxy, carboxaldéhyde, carboxamide, cycloalkyle, cycloalkényle, cycloalkynyle, cycloalkylalkyle, aryle, aroyle, aryloxy, arylamino, biaryle, thioaryle, diarylamino, hécérocyclyle, alkylaryle, aralkényle, aralkyle, alkylhétérocyclyle, hétérocyclylalkyle, sulfonyle, -SO₂-(alkyle en C₁-C₃), -SO₃- (alkyle en C₁-C₃), sulfonamido, aryloxyalkyle, carboxyle, carbamate et -C(O)NH(benzyle) ;
où si L est -NR¹³, R⁴ et R¹³ conjointement peuvent former un cycle ;
et où R⁶ et R⁸ conjointement peuvent former un cycle ;
et où R⁹ et R¹⁰ conjointement peuvent former un cycle ;
ou un sel pharmaceutiquement acceptable de ceux-ci.

2. Composé selon la revendication 1 qui est un dérivé de celui-ci choisi parmi les esters, les carbamates et les aminals.

3. Composé de structure 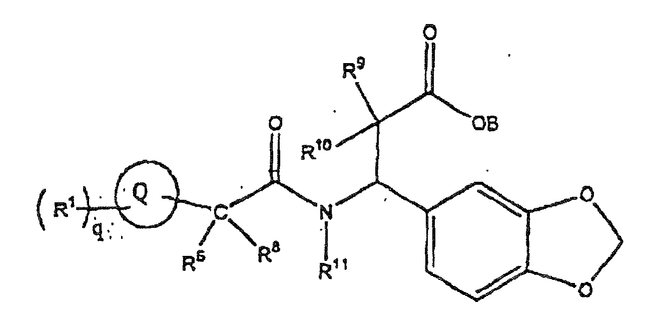 où le cercle Q est un cycle, constitué de 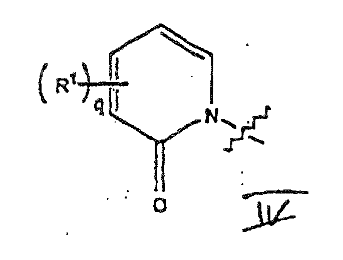
q est un nombre entier de zéro à quatre ; et
B, R¹, R⁶, R⁸, R⁹, R¹⁰ et R¹¹ sont respectivement choisis indépendamment dans le groupe constitué de l'hydrogène, l'halogène, les groupes hydroxyle, alkyle, alkényle, alkynyle, alcoxy, alkénoxy, alkynoxy, thioalcoxy, hydroxyalkyle, acyle aliphatique, -CF₃, nitro, amino, cyano, carboxy, -N(alkyle en C₁-C₃)-C(O) (alkyle en C₁-C₃), -NHC(O)NH(alkyle en C₁-C₃),-NHC(O)N(alkyle en C₁-C₃)C(O)NH(alkyle en C₁-C₃), -alkylamino en C₁-C₃, alkénylamino, alkynylamino, di(alkyle en C₁-C₃)amino, - C(O)O-(alkyle en C₁-C₃), -C(O)NH-(alkyle en C₁-C₃), -CH=NOH, - PO₃H₂, -OPO₃H₂, -C (O)N (alkyle en C₁-C₃)₂, haloalkyle, alcoxyalcoxy, carboxaldéhyde, carboxamide, cycloalkyle, cycloalkényle, cycloalkynyle, cycloalkylalkyle, aryle, aroyle, aryloxy, arylamino, biaryle, thioaryle, diarylamino, hétérocyclyle, alkylaryle, aralkényle, aralkyle, alkylhétérocyclyle, hétérocyclylalkyle, sulfonyle, -SO₂-(alkyle en C₁-C₃), -SO₃-(alkyle en C₁-C₃), sulfonamido, aryloxyalkyle, carboxyle, carbamate et -C(O)NH(benzyle) ;
où R⁶ et R⁸ conjointement peuvent former un cycle ;
et où R⁹ et R¹⁰ conjointement peuvent former un cycle ;
ou un sel pharmaceutiquement acceptable de ceux-ci.

4. Composé selon la revendication 3 qui est un dérivé de celui-ci, choisi parmi les esters, les carbamates et les aminals.

5. Composé selon la revendication 3, dans lequel R⁶, R⁸, R⁹, R¹⁰ et R¹¹ sont respectivement choisis indépendamment dans le groupe constitué de l'hydrogène et du groupe alkyle, et R¹ à chaque occurrence, est choisi indépendamment dans le groupe constitué de l'hydrogène, du 2-thiénylméthyle, des groupes benzyle et méthyle.

6. Composé choisi dans le groupe constitué de l'acide (3S)-3-(1,3-benzodioxol-5-yl)-3-((2R,S)-2-(3-benzyl-5-méthyl-2-oxo-1(2H)-pyridinyl)hexanoylamino)propionique, de l'acide (3S)-3-(1,3-benzodioxol-5-yl)'-3-((2R,S)-2-(3-(3-chlorobenzyl)-5-méthyl-2-oxo-1(2H)-pyridinyl)hexanoylamino) propionique, de l'acide (3S)-3-(1,3-benzodioxol-5-yl)-3-(((2S)-2-(2-oxo-3-(phénylméthyl)-1(2H)-pyridinyl)hexanoyl) amino) propionique, de l'acide (3S)-3-(1,3-benzodioxol-5-yl)-3-((2-(3-chlorophényl)méthyl)-5-méthyl-2-oxo-1(2H)-pyridinyl) hexanoyl)amino)propionique et leurs sels pharmaceutiquement acceptables.

7. Composé selon la revendication 6 qui est un dérivé de celui-ci, choisi parmi les esters, les carbamates et les aminals.

8. Composition pharmaceutique comprenant :
un composé selon la revendication 1 ;
dans un véhicule pharmaceutiquement acceptable.

9. Utilisation d'un composé selon la revendication 1, pour préparer un médicament pour inhiber sélectivement la fixation de l'intégrine α₄β₁ chez un mammifère.
